# EUROPEAN PATENT APPLICATION

(11) **EP 1 840 126 A1**
(43) Date of publication of application: **03.10.2007**
(21) Application number: 06711559.2
(22) Date of filing: 11.01.2006
(51) Int. Cl.: C07D 401/14, A61K 31/454, A61K 31/4545, A61K 31/497, A61K 31/501, A61K 31/506, A61P 25/08, A61P 25/22, A61P 25/24, A61P 25/28, C07D 403/04, C07D 413/14, C07D 417/14

(54) **2-(CYCLIC AMINOCARBONYL)INDOLINE DERIVATIVE AND MEDICINAL COMPOSITION CONTAINING THE SAME**

(30) Priority: 12.01.2005 JP 2005005206
(71) Applicant: Dainippon Sumitomo Pharma Co., Ltd., Osaka 541-8524 (JP)
(72) Inventor: KONDO, Katsunori, oki-cho, Suita-shi, Osaka, 5640053 (JP); MASUMOTO, Kaoru, oki-cho, Suita-shi, Osaka, 5640053 (JP); KOHAYAKAWA, Hitoshi, oki-cho, Suita-shi, Osaka, 5640053 (JP)
(74) Representative: Webster, Jeremy Mark
(86) International application number: PCT/JP2006/300214
(87) International publication number: WO 2006/075619

(57) **Abstract**

A compound of the following formula (I): 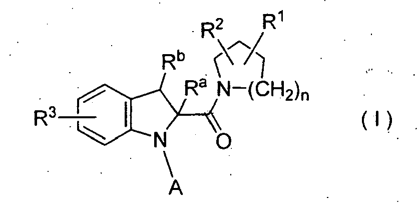 wherein A is a group of the following formula (I-A): 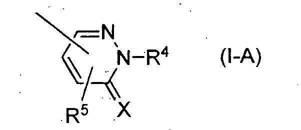 wherein X is an oxygen atom or a sulfur atom, R⁴ is a hydrogen atom, a C₁₋₆ alkyl group, or other, R⁵ is a hydrogen atom or other; or a heteroaryl group or other optionally substituted with a halogen, a C₁₋₆ alkyl, a C₁₋₆ alkoxy, or other; R¹ and R² are the same or different and are a hydrogen atom, a C₁₋₆ alkyl group or other; R³ is a hydrogen atom, a halogen atom, a C₁₋₆ alkoxy group, or other; R^{a} and R^{b} are the same or different and are a hydrogen atom or a C₁₋₆ alkyl group; and n is an integer of 0-5; or a pharmaceutically acceptable acid addition salt thereof, which can selectively act on mitochondrial benzodiazepine receptor and is useful as a medicament for treating/preventing anxiety disorder, depression, epilepsy, dementia, and so on.

## Description

### TECHNICAL FIELD

The present invention relates to a novel 2-(cyclic aminocarbonyl)indoline derivative which can selectively act on mitochondrial benzodiazepine receptor and a pharmaceutical composition containing thereof.

### BACKGROUND ART

The mitochondrial benzodiazepine receptor (hereinafter, optionally referred to as "MBR") is also called as a peripheral-type benzodiazepine receptor or an ω₃ receptor, and is known to be functionally and structurally different from a central-type benzodiazepine receptor (hereinafter, optionally referred to as "CBR") which forms a complex with γ-aminobutyric acid (hereinafter, optionally referred to as "GABA") A receptor and chloride ion channel. MBR widely exists in central nervous system, peripheral tissue (adrenal gland, testis, kidney, heart, lung, liver, smooth muscle and the like) and blood cells (erythrocyte, leukocyte, platelet), and is especially highly expressed in glandular tissue and secretory tissue. Although the physiological function of MBR has not fully been clarified, it has been reported that MBR may be implicated in steroidogenesis, apoptosis, cell proliferation, cell differentiation, immune functions, and mitochondrial functions. Additionally, MBR is an isoquinoline-binding protein of 18kDa and it forms a complex with voltage-dependent anion channel and adenine nucleotide translocase in mitochondria. This complex is referred to as a mitochondrial permeability transition pore.

In central nervous system, MBR is mainly located on mitochondrial outer membrane of glia cells, which relates to the transfer of cholesterol to mitochondrial inner membrane. The translocated cholesterol is metabolized to pregnenolone by cytochrome P450 cholesterol side-chain-cleavage enzyme in mitochondrial inner membrane, and further converted to various neurosteroids. The activity of cytochrome P450 cholesterol side-chain-cleavage enzyme depends on the amount of cholesterol supply and hence it is thought that MBR may modulate synthesis of neurosteroids. Therefore, a substance that can modulate MBR functions can modulate synthesis of neurosteroids.

Neurosteroids are known to act on various receptors and are related to various physiological functions. For example, allopregnanolone stimulates GABA A receptor complex and then suppresses excitability of cell, leading to antianxiety effect, anticonvulsant effect, sedative effect and so on; and dehydroepiandrosterone sulfate stimulates sigma receptor, leading to antidepressant effect. In addition, it is reported that a pregnenolone sulfate acts on NMDA receptor to have an effect on memory/learning function and neurosteroids such as progesterone promotes myelinogenesis.

Additionally it is known that the level of neurosteroids may vary depending on pathophysiological or physiological conditions. Rats exposed to a long-term isolation stress, i.e. a kind of chronic mild stress, show a decrease in neurosteroid levels. CB34, an MBR ligand, has been reported to increase the concentrations of neurosteroids to a greater extent in the rats exposed to this stress than in non-stressed rats, and thus it is suggested that the response of an MBR ligand might increase by chronic stress. It is also reported that a patient suffering from depression showed low concentration of allopregnanolone in cerebrospinal fluid and the level of neurosteroid was recovered in a patient whose condition of depression was improved by antidepressant.

FGIN-1-27 [chemical name: N,N-di-n-hexyl-2-(4-fluorophenyl)indol-3-acetamide] which is known as a selective MBR agonist enhances the neurosteroidogenosis, and exhibits antianxiety effect, anticonvulsant effect, antidepressant effect or cognitive enhancing effect in various animal models. Additionally, SSR180575 (chemical name: 7-chloro-N,N,5-trimethyl-4-oxo-3-phenyl-3,5-dihydro-4H-pyridazino[4,5-b]indol-1-acetamide) which exhibits a high affinity to MBR and enhances the neurosteroidogenosis has been found to reduce death of cells in an ischemia-reperfusion model, to have beneficial therapeutic effects in a rheumatoid arthritis model, and to promote neuronal protection and repair in an experimental model of motoneurone degeneration induced by facial nerve axotomy. Accordingly, a compound acting on MBR is useful as a medicament for treating anxiety disorders (panic disorder, generalized anxiety disorder, social-anxiety disorder, obsessive-compulsive disorder, posttraumatic stress disorder and so on), depressions/mood disorder, epilepsy, dementia (Alzheimer's disease, cerebrovascular dementia and so on). Furthermore, in addition to the above use, it is suggested that a compound which acts on MBR may be used as a treating and preventing agents for anxiety and depression, sleep disorder, nervous disease (Huntington's disease, multiple sclerosis, peripheral nerve disease and so on), stress-related gastrointestinal disorders (stomach and duodenal ulcer, irritable bowel syndrome and so on), inflammatory disease (rheumatoid arthritis and so on), cancer and so on.

WO96/32383 discloses acetamide derivatives which act on MBR. However, the compounds of the present invention are different from these acetamide derivatives at the viewpoint of a fundamental chemical structure.

WO 99/43672 discloses indoline compounds which exhibit inhibitory activity for phospholipase A₂. However, the chemical structure of the side chain in the indoline compound is really different from that of the present invention.

### DISCLOSURE OF INVENTION

### Problem to be solved by the invention

A problem to be solved by the invention is to provide a useful compound as a medicament for treating anxiety disorders and the relative disorders via selective and potent actions on MBR.

### Means to solve the problem

The present inventors have extensively studied on the above problem and have found a 2-(cyclic aminocarbonyl)indoline derivative of the formula (I) shown below which satisfies the object. Based upon the new findings, the present invention has been accomplished. The present invention provides a 2-(cyclic-aminocarbonyl)-indoliríe derivative of the following formula (I): 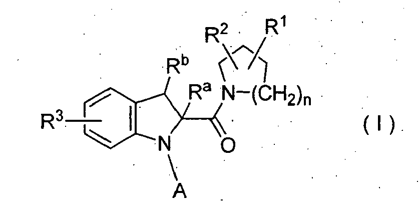 wherein
A is a group of the following formula (I-A): 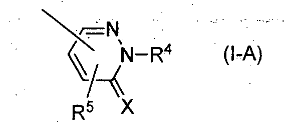 wherein X is oxygen atom or sulfur atom, R⁴ is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, a hydroxy-C₁₋₆ alkyl group, or a mono- or a di-fluoro-C₁₋₆ alkyl group, R⁵ is hydrogen atom or a halogen atom, or
   a heteroaryl group which is a 5- or a 6-membered monocyclic or fused polycyclic aromatic heteroaryl group containing 1-4 hetero atoms selected from the group consisting of N, O, and S, wherein the heteroaryl group may be optionally substituted with a halogen, a C₁₋₆ alkyl, a C₁₋₆ alkoxy group, nitro group or amino group;
R¹ and R² are the same or different and are hydrogen atom, a halogen atom, a C₁₋₆ alkyl group, trifluoromethyl group, a hydroxy-C₁₋₆ alkyl group, hydroxy group, amino group, a di (C₁₋₆ alkyl) amino group, a C₁₋₆ alkylcarbonylamino group, a (C₁₋₆ alkyl) (C₁₋₆ alkylcarbonyl) amino group, a C₁₋₆ alkyloxycarbonyl-amino group or an aryl group;
R³ is hydrogen atom, a halogen atom, a C₁₋₆ alkyl group, a C₂₋₆ alkenyl group, a C₁₋₆ alkoxy group, a hydroxy group, amino group, a di(C₁₋₆ alkyl)amino group, 1-pyrrolidinyl group, 1-piperidinyl group, an aryl-C₁₋₆ alkylamino group or an aryl group;
R^{a} and R^{b} are the same or different and are hydrogen atom or a C₁₋₆ alkyl group; and
n is an integer of 0-5,
and a pharmaceutically acceptable acid addition salt thereof (hereinafter, optionally referred to as "the compound of the present invention").

The pharmaceutically acceptable acid addition salt of the compound of the formula (I) means a pharmaceutically acceptable acid addition salt of the compound of the formula (I) which has a sufficient basicity to form an acid addition salt, and includes, for example, a mineral acid salt such as hydrochloride, hydrobromide, hydroiodide, sulfate, and phosphate; an organic acid salt such as oxalate, malonate, maleate, fumarate, lactate, malate, citrate, tartrate, benzoate, trifluoroacetate, acetate, methanesulfonate, p-toluenesulfonate, and trifluoromethanesulfonate; an amino acid salt such as glutamate and aspartate.

The compound of the formula (I) and an acid addition salt thereof may exist as a form of hydrate and/or solvate and then the hydrate and/or solvate form thereof is also within the scope of the invention. In addition, the compound of the formula (I) may exist as several stereoisomers since the compound has one or more asymmetric carbon atoms. In addition, the compound of the formula (I) may exist as a tautomer. These stereoisomers, a mixture thereof and a racemic compound thereof are contemplated herein. The compound of the formula (I) which is replaced by one or more radioactive isotopes is also contemplated herein.

The terms used herein are illustrated as follows.

The term "C₁₋₆ alkyl group" may be either a straight or a branched chain and includes, for example, methyl group, ethyl group, propyl group, isopropyl group, butyl group, isobutyl group, sec-butyl group, tert-butyl group, pentyl group, isopentyl group, hexyl group, and the like.

The term "C₂₋₆ alkenyl group" may be either a straight or a branched chain having at least one double bond and includes, for example, vinyl group, allyl group, 1-propenyl group, isopropenyl group, 1-, 2-, or 3-butenyl group, 2-, 3-, or 4-pentenyl group, 2-methyl-2-butenyl group, 3-methyl-2-butenyl group, 5-hexenyl group, and the like.

The examples of "C₃₋₆ cycloalkyl group" are cyclopropyl group, cyclobutyl group, cyclopentyl group, and cyclohexyl group and the like.

The term "C₃₋₆ cycloalkyl-C₁₋₆ alkyl group" means a "C₁₋₆ alkyl group" substituted with a "C₃₋₆ cycloalkyl group" and includes, for example, cyclopropylmethyl group, cyclobutylmethyl group, cyclopentylmethyl group, cyclohexyl-methyl group and the like.

The term "aryl group" means phenyl group or a fused polycyclic aromatic hydrocarbon group comprising benzene ring(s) and includes, for example, phenyl group, naphthyl group and the like.

The term "aryl-C₁₋₆ alkyl group" means a "C₁₋₆ alkyl group" substituted with an "aryl group" and includes, for example, benzyl group, phenethyl group, phenylpropyl group, naphthylmethyl group, and the like.

The term "arylcarbonyl-C₁₋₆ alkyl group" means a "C₁₋₆ alkyl group" substituted with an "arylcarbonyl group" and includes, for example, phenylcarbonylmethyl group, phenyl-carbonylethyl group, naphthylcarbonylmethyl group, and the like.

"C₁₋₆ alkoxy" may be,either a straight or a. branched chain and includes, for example, methoxy, ethoxy, propoxy, isopropoxy, butoxy, isobutoxy, sec-butoxy, tert-butoxy, and the like.

The term "hydroxy-C₁₋₆ alkyl" means a "C₁₋₆ alkyl group" substituted with hydroxy group and includes, for example, hydroxymethyl group, hydroxyethyl group, hydroxypropyl group, hydroxybutyl group, hydroxypentyl group, hydroxyl-isopentyl group, hydroxyhexyl group, and the like.

The term "mono- or di-fluoro-C₁₋₆ alkyl group" means a "C₁₋₆ alkyl group" substituted with 1 or 2 fluorine atoms and includes, for example, fluoromethyl group, 2-fluoroethyl group, 3-fluoropropyl group, difluoromethyl group, 2,2-difluoroethyl group, and the like.

The term "di(C₁₋₆ alkyl)amino group" means amino group substituted with two same or different "C₁₋₆ alkyl groups" and includes, for example, dimethylamino group, diethyl-amino group, dipropylamino group, ethylmethylamino group, and the like.

The term "C₁₋₆ alkylcarbonylamino group" means amino group substituted with a "C₁₋₆ alkylcarbonyl group" and includes, for example, acetylamino-group, ethylcarbonyl-amino group, isopropylcarbonylamino group, and the like.

The term "(C₁₋₆ alkyl) (C₁₋₆ alkylcarbonyl) amino group" means amino group substituted with a "C₁₋₆ alkyl group" and a "C₁₋₆ alkylcarbonyl group" and includes, for example, acetylmethylamino group, acetylethylamino group, and the like.

The term "aryl-C₁₋₆ alkylamino group" means amino group substituted with an "aryl-C₁₋₆ alkyl group" and includes, for example, benzylamino group, phenethylamino group, phenyl-propylamino group, naphthylmethylamino group, and the like.

The term "C₁₋₆ alkyloxycarbonylamino group" means amino group substituted with a "C₁₋₆ alkyloxycarbonyl" and includes, for example, methyloxycarbonylamino group, ethyloxycarbonylamino group, propyloxycarbonylamino group, tert-butyloxycarbonylamino group, and the like.

The term "heteroaryl group which is a 5- or a 6-membered monocyclic or fused polycyclic aromatic heteroaryl group containing 1-4 hetero atoms selected from the group consisting of N, O, and S" means a heteroaryl group which is denoted as a 5- or a 6-membered monocyclic unsaturated hydrocarbon group or a fused polycyclic unsaturated hydrocarbon thereof wherein 1-4 carbon atoms of the ring is displaced by hetero atoms selected from the group consisting of N, O, and S, and the examples thereof are furyl group, thienyl group, oxazolyl group, isoxazolyl group, thiazolyl group, isothiazolyl group, pyridyl group, pyrazinyl group, pyrimidinyl group, pyridazinyl group, benzoxazolyl group, benzisoxazolyl group, benzothiazolyl group, benzisothiazolyl group, quinolyl group, isoquinolyl group, quinoxalinyl group, quinazolinyl group, phthalazinyl group; cinnolinyl group, naphthyridinyl group, imidazopyridazinyl group, triazolopyridazinyl group, and the like. The preferable examples thereof are thiazolyl group, pyridyl group, pyrazinyl group, pyrimidinyl group, pyridazinyl group, 1,3-benzoxazolyl group, 1,3-benzothiazolyl group, 1,2-benzisothiazolyl group, isoquinolyl group, quinoxalinyl group, imidazo[1,2-b]pyridazinyl group, and triazolo[4,3-b]pyridazinyl group; more preferably, 2-thiazolyl group, 2-pyridyl group, 3-pyridyl group, 4-pyridyl group, 2-pyrazinyl group, 2-pyrimidinyl group, 4-pyrimidinyl group, 3-pyridazinyl group, 1,3-benzoxazol-2-yl group, 1,3-benzothiazol-2-yl group, 1,2-benzisothiazol-3-yl group, 1-isoquinolyl group, 2-quinoxalinyl group, 6-imidazo[1,2-b]pyridazinyl group, and 1, 2, 4-triazolo[4, 3-b]pyridazin-6-yl group; even more preferably 2-pyrazinyl group, 2-pyrimidinyl group, 3-pyridazinyl group, 1, 3-benzoxazol-2-yl group, 6-imidazo[1, 2-b]pyridazinyl group, and 6-[1, 2, 4]triazolo[4, 3-b]pyridazinyl group; and most preferably 2-pyrazinyl group, 3-pyridazinyl group, 1,3-benzoxazol-2-yl group, 6-imidazo[1, 2-b]pyridazinyl group, and 1, 2, 4-triazolo[4, 3-b]pyridazin-6-yl group.

The term "halogen atom" means fluorine atom, chlorine atom, bromine atom, or iodine atom.

The preferable compounds in the present invention are 2-(cyclic aminocarbonyl)indoline derivatives of the formula-(I) or pharmaceutically acceptable acid addition salts thereof, wherein R¹ and R² are the same or different and are hydrogen atom, a C₁₋₆ alkyl group or trifluoromethyl group; R³ is a hydrogen atom, a halogen atom, a C₁₋₆ alkyl group, a C₂₋₆ alkenyl group or a C₁₋₆ alkoxy group; R^{a} is hydrogen atom; A, R^{b}, and n are as defined above.

The more preferable compounds are 2-(cyclic aminocarbonyl)indoline derivatives of the formula (I), wherein
A is a group of the following formula (I-A1): 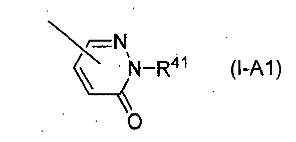 wherein R⁴¹ is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, or a mono- or a di-fluoro-C₁₋₆ alkyl group, or
   furyl group, thienyl group, oxazolyl group, isoxazolyl group, thiazolyl group, isothiazolyl group, pyridyl group, pyrazinyl group, pyrimidinyl group, pyridazinyl group, benzoxazolyl group, benzisoxazolyl group, benzothiazolyl group, benzisothiazolyl group, quinolyl group, isoquinolyl group, quinoxalinyl group, quinazolinyl group, phthalazinyl group, cinnolinyl group, naphthyridinyl group, imidazopyridazinyl group or triazolopyridazinyl group, wherein each group may be optionally substituted with a halogen atom, a C₁₋₆ alkyl group or a C₁₋₆ alkoxy group;
R¹ and R² are the same or different and are hydrogen atom, a C₁₋₆ alkyl group or trifluoromethyl group;
R³ is hydrogen atom, a halogen atom, a C₁₋₆ alkyl group, a C₂₋₆ alkenyl group or a C₁₋₆ alkoxy group;
R^{a} is, hydrogen atom;
R^{b} is hydrogen atom or a C₁₋₆ alkyl group; and
n is an integer of 1-4,
or pharmaceutically acceptable acid addition salts thereof.

The even more preferable compounds are 2-(cyclic aminocarbonyl)indoline derivatives of the formula (I), wherein
A is a group of the following formula (I-A2): 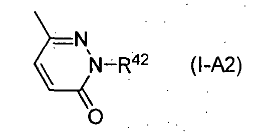 wherein R⁴² is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, or a mono- or a di-fluoro-C₁₋₆ alkyl group, or
a group of the following formula (I-A3): 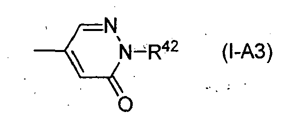 wherein R⁴² is as defined above;
R¹ and R² are the same or different and are hydrogen atom, a C₁₋₆ alkyl group or trifluoromethyl group;
R³ is hydrogen atom, a halogen atom or a C₁₋₆ alkoxy group;
R^{a} is hydrogen atom;
R^{b} is hydrogen atom or methyl group; and
n is an integer of 1-4,
or pharmaceutically acceptable acid addition salts thereof.

The even more preferable compounds are 2-(cyclic aminocarbonyl)indoline derivatives of the formula (I), which have the following formula (Ia): 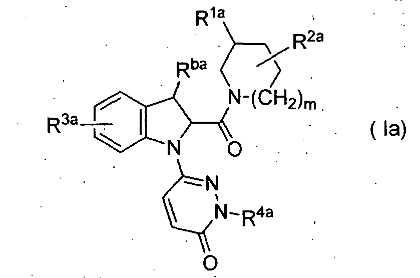 wherein
R^{1a} is hydrogen atom, methyl group, ethyl group, propyl group or trifluoromethyl group, which is bound on the 3-positions of the cyclic amino group;
R^{2a} is hydrogen atom or methyl group, which is bound on the 3- or 5-positions of the cyclic amino group;
R^{3a} is hydrogen atom, fluorine atom, chlorine atom, bromine atom, or methoxy group, which is bonded on the 4-, 5-, or 6-position of the indoline ring;
R^{4a} is hydrogen atom, methyl group, ethyl group, propyl group, isopropyl group, butyl group, isobutyl group, sec-butyl group, cyclopentyl group, cyclohexylmethyl-group, benzyl group, phenylcarbonylmethyl group, 2-fluoroethyl group or 2,2-difluoroethyl group;
R^{ba} is hydrogen atom or methyl group; and
m is 0, 1, 2 or 3,
or pharmaceutically acceptable acid addition salts thereof.

The still more preferable compounds are 2-(cyclic aminocarbonyl)indoline derivatives of the formula (I), wherein
A is thiazolyl group, pyridyl group, pyrazinyl group, pyrimidinyl group, pyridazinyl group, 1,3-benzoxazolyl group, 1,3-benzothiazolyl group, 1,2-benzisothiazolyl group, isoquinolyl group, quinoxalinyl group, imidazo[1,2-b]pyridazinyl group, or 1,2,4-triazolo[4,3-b]pyridazinyl group, wherein each group may be optionally substituted with a halogen atom, a C₁₋₆ alkyl- group or a C₁₋₆ alkoxy group;
R¹ and R² are the same or different and are hydrogen atom, C₁₋₆ alkyl group or trifluoromethyl group;
R³ is hydrogen atom, a halogen atom or a C₁₋₆ alkoxy group;
R^{a} is hydrogen**·** atom;
R^{b} is hydrogen atom or methyl group; and
n is an integer of 1-4,
or pharmaceutically acceptable acid addition salts thereof.

The still more preferable compounds are 2-(cyclic aminocarbonyl)indoline derivatives of the formula (I), wherein

A is 2-thiazolyl group, 2-pyridyl group, 3-pyridyl group, 4-pyridyl group, 2-pyrazinyl group, 2-pyrimidinyl group, 4-pyrimidinyl group, 3-pyridazinyl group, 1,3-benzoxazol-2-yl group, 1,3-benzothiazol-2-yl group, 1,2-benzisothiazol-3-yl group, 1-isoquinolyl group, 2-quinoxalinyl group, or 1,2,4-triazolo[4,3-b]pyridazin-6-yl group, wherein each group may be optionally substituted with fluorine, chlorine, methyl, ethyl or methoxy;
R¹ and R² are the same or different and are a hydrogen atom, methyl group, or trifluoromethyl group, wherein R¹ bound on the 3-positions of the cyclic amino group, and R², bound on the 3- or 5-positions of the cyclic amino group;
R³ is hydrogen atom, fluorine atom, chlorine atom, bromine atom, or methoxy group, which is bound on the 4-, 5-, or 6-position of the indoline ring;
R^{a} is hydrogen atom;
R^{b} is hydrogen atom or methyl group; and
n is 1, 2, 3, or 4,
or pharmaceutically acceptable acid addition salts thereof.

The preferable compounds are the compounds of the formula (I), wherein the configuration of the 2-positioned asymmetric carbon in the indoline ring is (S).

The preferable examples of the compounds of the formula (I) are
(S)-1-(1,3-benzoxazol-2-yl)-2-[[(S)-3-methylpiperidin-1-yl]carbonyl]indoline (the compound of Example 17),
(S)-2-[(S)-3-methylpiperidin-1-yl]carbonyl-1-(2-pyrimidinyl)indoline (the compound of Example 23),
(S)-2-(cis-3,5-dimethylpiperidin-1-yl)carbonyl-1-(2-pyrimidinyl)indoline (the compound of Example 24),
(S)-1-(1-methyl-6-oxo-1,6-dihydropyridazin-3-yl)-2-[[(S)-3-methylpiperidin-1-yl]carbonyl]indoline (the compound of Example 40),
(S)-1-(1-methyl-6-oxo-1,6-dihydropyridazin-3-yl)-2-[(cis-3,5-dimethylpiperidin-1-yl)carbonyl]indoline (the compound of Example 41),
(S)-2-(cis-3,5-'dimethylpiperidin-1-yl)carbonyl-1-(3-pyridazinyl)indoline (the compound of Example 57),
(S)-2-[(S)-3-methylpiperidin-1-yl]carbonyl-1-(2-pyrazinyl)indoline (the compound of Example 60),
(S)-2-(cis-3,5-dimethylpiperidin-1-yl)carbonyl-1-(2-pyrazinyl)indoline (the compound of Example 61),
(S)-2-[(S)-3-methylpiperidin-1-yl]carbonyl-1-(3-, pyridazinyl)indoline (the compound of Example 63),
(S)-1-(1-ethyl-6-oxo-1,6-dihydropyridazin-3-yl)-2-[[(S)-3-methylpiperidin-1-yl]carbonyl]indoline (the compound of Example 129),
(S)-1-(1-methyl-6-oxo-1,6-dihydropyridazin-3-yl)-2-[(1-perhydroazepinyl)carbonyl]indoline (the compound of Example 118),
(S)-1-(1-methyl-6-oxo-1,6-dihydropyridazin-3-yl)-2-[(1-perhydroazocinyl)carbonyl]indoline (the compound of Example 119),
(S)-1-(3-chloro-1,2,4-triazolo[4,3-b]pyridazin-6-yl)-2-[[(S)-3-methylpiperidin-1-yl]carbonyl]indoline (the compound of Example 79),
(S)-1-[1-(2-fluoroethyl)-6-oxo-1,6-dihydropyridazin-3-yl]-2-[[(S)-3-methylpiperidin-1-yl]carbonyl]indoline (the compound of Example 128),
(S)-2-[[(S)-3-methylpiperidin-1-yl]carbonyl]-1-(3-methyl-1,2,4-triazolo[4,3-b]pyridazin-6-yl)indoline (the compound of Example 80),
(S)-2-[[(S)-3-methylpiperidin-1-yl]carbonyl]-1-(1,2,4-triazolo[4,3-b]pyridazin-6-yl)indoline (the compound of Example 154), and
(S)-2-[[(S)-3-methylpiperidin-1-yl]carbonyl]-1-(6-oxo-1,6-dihydropyridazin-3-yl)indoline (the compound of Example 143),
or pharmaceutically acceptable acid addition salts thereof.

The compounds of the formula (I) can be prepared, for example, according.to the following method.

### Process A

The compound of the formula (I) wherein R^{a} is a hydrogen atom, except for the compound wherein A is a heteroaryl group substituted with amino group or a hydroxyl group, the compound wherein R¹ or R² is a hydroxy-C₁₋₆ alkyl group, hydroxy group, amino group or a C₁₋₆ alkyloxycarbonylamino group, and the compound wherein R³ is hydroxy group or amino group, can be prepared by reacting the compound of the following formula (II): 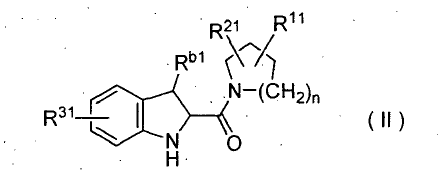 wherein R¹¹ and R²¹ are the same or different and are hydrogen atom, a halogen atom, a C₁₋₆ alkyl group, trifluoromethyl group, a di (C₁₋₆ alkyl)amino group, a C₁₋₆ alkylcarbonyl-amino group, a (C₁₋₆ alkyl) (C₁₋₆ alkylcarbonyl)-amino group, or an aryl group; R³¹ is hydrogen atom, a halogen atom, a C₁₋₆ alkyl group, a C₁₋₆ alkoxy group, or an aryl group; R^{b1} is hydrogen atom or a C₁₋₆ alkyl group; and n is an integer of 0-5, and the compound of the following formula (III):

A¹-Z (III)

wherein A¹ is the group as defined in the formula (I-A), except for the group wherein R⁴ is a hydroxy-C₁₋₆ alkyl group; or a 5- or a 6-membered monocyclic or fused polycyclic aromatic heteroaryl group containing 1-4 hetero atoms selected from the group consisting of N, O, and S, wherein the heteroaryl group may be optionally substituted with a halogen atom, a C₁₋₆ alkyl group, a C₁₋₆ alkoxy group or a nitro group; and Z is a halogen atom.

The reaction between the compound of the formula (II) and the compound of the formula (III) can be carried out in the presence of a base in an appropriate solvent. The reaction can be also carried out in the presence of a palladium catalyst, a phosphine and a base in an appropriate solvent. The palladium catalyst includes, for example, tris(dibenzylideneacetone)dipalladium(0), bis(dibenzylideneacetone)palladium(0), palladium(II) acetate and the like, preferably tris(dibenzylideneacetone)dipalladium. The phosphine include, for example, tri-tert-butyl phosphine, 2-(di-tert-butyl phosphino)-biphenyl, 2-(dicyclohexylphosphino)biphenyl and the like. The base includes, for example, an inorganic base such as potassium carbonate, sodium carbonate, barium carbonate, cesium carbonate, sodium hydrogen carbonate, potassium hydrogen carbonate, sodium hydroxide, potassium hydroxide, lithium hydroxide, and barium hydroxide; an organometallic base such as sodium tert-butoxide, sodium tert-pentoxide, potassium tert-butoxide, lithium tert-butoxide, sodium methoxide, sodium ethoxide, sodium isopropoxide, sodium hydride, lithium hexamethyl disilazide, sodium hexamethyl disilazide, potassium hexamethyl disilazide, lithium diisopropylamide, sodium lithium diisopropylamide, potassium lithium diisopropylamide, n-butyl lithium, sec-butyl lithium, tert-butyl lithium and the like; an organic base such as triethylamine, diisopropylethylamine, 2,2,6,6-tetramethylpiperidine, pyridine, 1,8-diazabicyclo[5.4.0]undec-7-ene, 1,5-diazabicyclo[4.3.0]non-5-ene, 1,4-diazabicyclo[2.2.2]octane and the like. The preferable examples of the base are sodium hexamethyldisilazide, sodium tert-butoxide; potassium tert-butoxide, lithium tert-butoxide, triethylamine, and diisopropylethylamine. In using an inorganic base or an organometallic base, a crown ether such as 18-crown-6 may be occasionally added thereto. The solvent includes, for example, an aromatic hydrocarbon such as toluene and xylene and the like; ethers such as tetrahydrofuran, 1,4-dioxane and diethylene glycol dimethyl ether (1,2-dimethoxyethane) and the like; lower alcohols such as methanol, ethanol, propanol, butanol, and tert-butanol and the like; ethyl acetate, acetone, acetonitrile, pyridine, dimethyl sulfoxide, dimethyl formamide, and N-methylpyrrolidone. These solvents may be used in single or as a mixture of two or more solvents. The reaction temperature is generally about -20°C to about 150°C, preferably about 0°C to about 100°C.

The compound of the formula (II) wherein R^{b1} is hydrogen atom can be prepared, for example, by a method as shown in the following scheme. 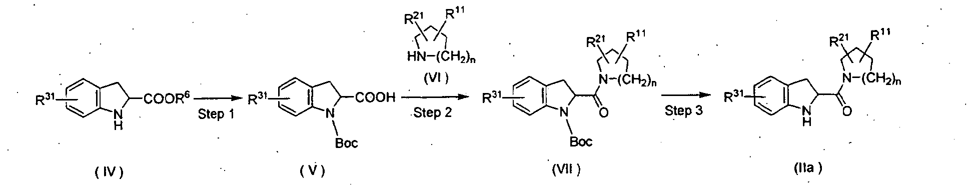 wherein Boc is tert-butoxycarbonyl; R⁶ is hydrogen atom or a lower alkyl group; R^{11,} R²¹, R³¹ and n are as defined above.

### (Step 1)

The compound of the formula (V) can be prepared by reacting the compound of the formula (IV) and Boc reagent [e.g. tert-butoxycarbonyl chloride, di-tert-butyl dicarbonate; N-(tert-butoxycarbonyloxy)phthalimide, 1-(tert-butoxy-carbonyl)-1,2,4-triazole, 2-(tert-butoxy-carbonyloxyimino)-2-phenylacetonitrile and the like] in an appropriate solvent, optionally adding a base. The examples of the base are sodium carbonate, potassium carbonate, sodium hydrogen carbonate, sodium hydroxide, potassium hydroxide, triethylamine, diisopropylethylamine and the like. The examples of the solvent are 1,4-dioxane, tetrahydrofuran, acetonitrile, acetone, chloroform, dichloromethane, tert-butanol, water and the like. These solvents may be used in single or as a mixture of two or more solvents. The reaction temperature is generally about -20°C to about 100°C, preferably about 0°C to about 40°C.

### (Step 2)

The compound of the formula (VII) can be prepared through dehydration-condensation between the compound of the formula (V) and the compound of the formula (VI) or an acid addition salt thereof. The dehydration-condensation may be carried out according to known dehydration-condensation between secondary amines and carboxylic acids or similar method thereof. For example, the compound of the formula (V) and the compound of the formula (VI) are dehydrated/condensed using a condensing agent such as N,N'-dicyclohexylcarbodiimide, 1-ethyl-3-(3-dimethylaminopropyl)carbodiimide, 1-ethyl-3-(3-dimethylaminopropyl)-carbodiimide hydrochloride, 1,1'-carbonyl-bis-1H-imidazole (a.k.a: N,N'-carbonyldiimidazole), N,N'-carbonyl-disuccinimide, 1-ethoxycarbonyl-2-ethoxy-1,2-dihydroquinoline, diphenylphosphoryl azide, propanephosphonic anhydride, benzotriazol-1-yloxy-tris(dimethylamino)-phosphonium·hexafluorophosphate, benzotriazol-1-yloxy-tris(pyrrolidino)phosphonium·hexafluoro-phosphate, 1-tert-butoxy-2-tert-butoxycarbonyl-1,2-dihydroisoquinoline; with or without an activator (1-hydroxyberizotriazole, N-hydroxysuccinimide, 3,4-dihydro-3-hydroxy-4-oxo-1,2,3-benzotriazine and the like); in an appropriate solvent. The solvent includes, for example, aromatic hydrocarbons such as toluene, xylene; ethers such as diethyl ether, tetrahydrofuran, 1,4-dioxane; halogenated hydrocarbons such as dichloromethane and chloroform; esters such as ethyl acetate; dimethylformamide; or the like, these solvents may be used in single or as a mixture of two or more solvents. The reaction temperature may vary depending on the type of the starting compound, generally about -30°C to about 100°C, preferably about -10°C to about 40°C.

### (Step 3)

The compound of the formula (IIa) can be prepared by contacting the compound of the formula (VII) and an acid in an appropriate solvent. The acid includes, for example, trifluoroacetic acid, hydrochloric acid, hydrobromic acid, sulfuric acid, oxalic acid, acetic acid, p-toluenesulfonic acid, methanesulfonic acid, trifluoromethanesulfonic acid and the like. The solvent includes, for example, chloroform, dichloromethane, dichloroethane, tetrahydrofuran, 1,4-dioxane, toluene, xylene, ethanol, methanol, ethyl acetate, and water. These solvents may be used in single or as a mixture of two or more solvents. The reaction temperature is typically about -30°C to about 100°C, preferably about 0°C to about 40°C.

The compound of the formula (II) can be also prepared, for example, by a method as shown in the following scheme. 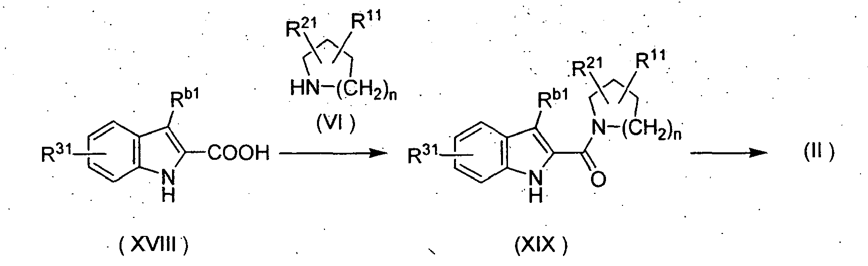 Wherein R¹¹, R²¹, R³¹, R^{b1} and n are as defined above.

The compound of the above formula (XIX) can be prepared according to the process of the compound of the above formula (VII) or similar process thereof. The compound of the above formula (II) can be prepared through a method reducing the compound of the formula (XIX) with magnesium in methanol as a solvent ; a method reducing it with a reducing agent such as sodium borohydride and sodium cyanoborohydride in a solvent such as acetic acid and trifluoroacetic acid; or a method reducing it with a catalyst such as platinum, palladium and Raney nickel under atmospheric or pressured hydrogen in an appropriate solvent.

The optically active compound of the formula (I) can be prepared using the corresponding optically active compounds (III), (IV) and (VI) as a starting material.

The compound of the formula (III) is commercially available or can be prepared through known methods or modified methods thereof. For example, 2-alkyl-6-chloro-2H-pyridazin-3-one can be prepared through the methods of Chem. Pharm. Bull., 35, 350-356 (1987) or Heterocycles, 29, 67-77 (1989), or similar methods thereof. 6-Chloro-imidazo[1,2-b]pyridazine can be prepared through the method of Bioorg. Med. Chem. Lett., 14, 2249-2252 (2004).

The compound of the formula (IV) is commercially available or can be prepared through known methods or modified methods thereof. It can be prepared through the methods of for example, J. Med. Chem., 26, 394-403 (1983), Bull. Korean Chem. Soc., 8, 434-435 (1987), JP-A-2-191251, WO99/33801, or similar methods thereof.

The compound of the formula (VI) is commercially available or can be prepared through known methods or modified methods thereof. For example, optically active 3-methylpiperidine can be prepared through the method of Naunyn-Schmiedeberg's Arch. Pharmacol., 315, 203-209 (1981) or similar methods thereof; 3,5-cis-dimethylpiperidine hydrochloride can be prepared through the method of J. Chem. Soc., Perkin Trans. 2, 1972; 1846-1853; pyrrolidine, piperidine and hexamethyleneimine substituted with alkyl group can be prepared through the method of Tetrahedron Lett., 35, 2529-2532 (1994) or similar methods thereof.

The compound of the formula (XVIII) is commercially available or can be prepared through known methods or modified methods thereof.

### Process B

The compound of the formula (I), except for the compound wherein A is a heteroaryl group substituted with amino group, the compound wherein at least one of R¹ and R² is an amino group, and the compound wherein R³ is hydroxy group or an amino group, can be prepared through dehydration-condensation between the compound of the following formula (VIII): 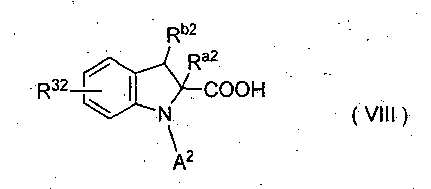 wherein A² is the group as defined in the formula (I-A); or a 5- or 6-membered monocyclic or fused polycyclic aromatic heteroaryl group containing 1-4 hetero atoms selected from the group consisting of N, O, and S, wherein the heteroaryl group may be optionally substituted with a halogen atom, a C₁₋₆ alkyl group, a C₁₋₆ alkoxy group or nitro group; R³² is hydrogen atom, a halogen atom, a C₁₋₆ alkyl group, a C₂₋₆ alkenyl group, a C₁₋₆ alkoxy group, a di (C₁₋₆ alkyl) amino group, 1-pyrrolidinyl group, 1-piperidinyl group or an aryl group; and R^{a2} and R^{b2} are the same or different and are a hydrogen atom or a C₁₋₆ alkyl group, and the compound of the following formula (IX): 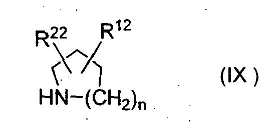 wherein
R¹² and R²² are the same or different and are hydrogen atom, a halogen atom, a C₁₋₆ alkyl group, trifluoromethyl group, a hydroxy-C₁₋₆ alkyl group, a hydroxy group, a di(C₁₋₆ alkyl) amino group, a C₁₋₆ alkylcarbonylamino group, a (C₁₋₆ alkyl) (C₁₋₆ alkyl-carbonyl) amino group, a C₁₋₆ alkyloxycarbonylamino group or an aryl group; and n is an integer of 0-5, or an acid addition salt thereof.

The reaction between the compound of the formula (VIII) and the compound of the formula (IX) can be carried out as mentioned in Step 2 of Process A.

The compound of the formula (IX) is commercially available or can be prepared through known methods or modified methods thereof as well as the compound of the above formula (VI).

The compound of the formula,(VIII) wherein R^{a2} is hydrogen atom can be prepared for example, by hydrolyzing the compound of the following formula (X): 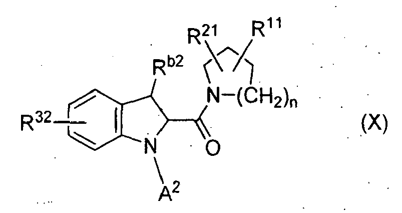 wherein A², R¹¹, R²¹, R³², R^{b2} and n are as defined above, which can be prepared through Process A, using a conventional method.

The compound of the formula (VIII) wherein A² is the above formula (I-A2) can be also prepared, for example through the following routes (Route A and Route B). 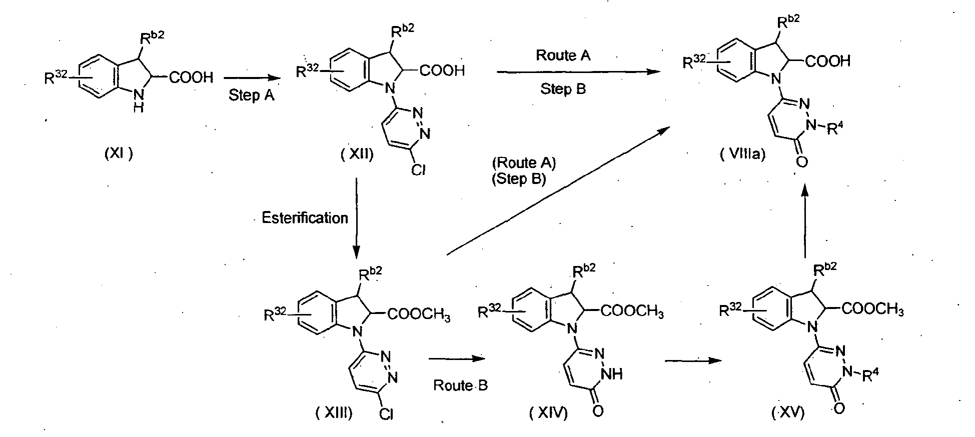 Wherein R³², R⁴ and R^{b2} are as defined above.

### (Step A)

The compound of the formula (XII) can be prepared by reacting the compound of the formula (XI) and 3,6-dichloropyridazine according to the above Process A. The reaction temperature is generally about -20°C to about 100°C, preferably about 0°C to about 60°C.

### (Route A/Step B)

The compound of the formula (VIIIa) can be prepared by reacting a quaternary salt which is obtained by reacting the compound of the formula (XII) or the compound of the formula (XIII) and an alkylating agent, with an appropriate alkaline aqueous solution and then making the reaction mixture to acidic with an appropriate acid. The examples of the alkylating agent include an alkyl halide such as methyl iodide and ethyl iodide and the like; a dialkyl sulfate such as dimethyl sulfate, diethyl sulfate and dipropyl sulfate and the like; methyl trifluoromethanesulfonate, trimethyl oxonium tetrafluoroborate, trimethylsulfoxonium iodide, dimethyl carbonate, and so on. The solvent includes aromatic hydrocarbons such as toluene, xylene; ethers such as tetrahydrofuran, 1,4-dioxane, and 1,2-dimethoxyethane; lower alcohols such as methanol, ethanol, butanol and tert-butanol and the like; ethyl acetate, acetone, acetonitrile, dimethyl sulfoxide, dimethylformamide, dimethylacetamide, N-methylpyrrolidone. These solvents may be used in single or as a mixture of two or more solvents. The reaction temperature is generally about -20°C to about 100°C, preferably about 0°C to about 70°C. The alkaline aqueous solution includes aqueous sodium hydroxide, aqueous potassium hydroxide, aqueous potassium carbonate, aqueous sodium carbonate, aqueous sodium hydrogen carbonate, aqueous potassium hydrogen carbonate, aqueous ammonia and so on. The acid includes, for example, hydrochloric acid, sulfuric acid, citric acid, trifluoroacetic acid, and acetic acid, and so on.

### (Route B)

The compound of the formula (VIIIa) can be prepared by hydrolyzing the compound of the formula (XV) which is prepared through hydrolysis of the pyridazine ring of the compound of the formula (XIII) and alkylation of it, according to a conventional method.

The compound of the formula (XI) is commercially available or can be prepared through known methods or modified methods thereof.

The compound of the formula (VIII) wherein R^{a2} is C₁₋₆ alkyl group can be prepared according to a conventional method as shown in the following route, using as a starting material the compound of the formula (XVI) which is prepared according to the preparation method of the above 1-substituted indoline-2-carboxylic acids. 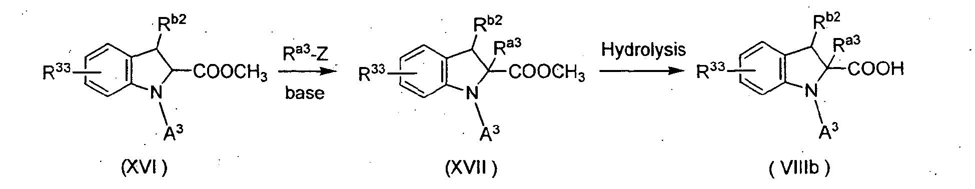 Wherein R^{a3} is a C₁₋₆ alkyl group; A³ is the group as defined in formula (I-A); or a 5- or a 6-membered monocyclic or fused polycyclic aromatic heteroaryl group containing 1-4 hetero atoms selected from the group consisting of N, O, and S, wherein the heteroaryl group may be optionally substituted with a C₁₋₆ alkyl, a C₁₋₆ alkoxy or a nitro; R³³ is hydrogen atom, a C₁₋₆ alkyl group, a C₂₋₆ alkenyl group, a C₁₋₆ alkoxy group, a di (C₁₋₆ alkyl) amino group, 1-pyrrolidinyl group, 1-piperidinyl group or an aryl group; and R^{b2} is hydrogen atom or a C₁₋₆ alkyl group; Z is a halogen atom.

The compound of the formula (I) wherein A is the above formula (I-A2) can be also prepared by the same process mentioned in the above (Route A/Step B), using the corresponding starting material, i.e., the substituent corresponding to A of the formula (I) is 6-chloropyridazin-3-yl group.

The compound of the formula (I) having an amino group on the substituent of A can be prepared by reducing the compound of the formula (I) having a nitro group on the corresponding position according to conventional reducing method. The reducing reaction can be carried out by reacting the compound of the formula (I) having a nitro group under atmospheric or pressured hydrogen in the presence of a catalyst. The catalyst includes, for example, platinum, palladium, Raney nickel and the like. The solvent includes, for example, ethyl acetate, methanol, ethanol, tetrahydrofuran, dimethylformamide, N-methylpyrrolidone, water and a mixture thereof. The reaction temperature is generally about 0°C to about 60°C.

When the benzene ring or heteroaryl in the compound of the formula (I) and the intermediate thereof is substituted with a halogen atom, the halogen atom can be exchanged for a hydrogen atom by a conventional reducing method. The reducing reaction may be carried out in the similar method to the above-mentioned reduction of nitro group, preferably at the presence of a base. The base includes, for example, sodium carbonate, potassium carbonate, sodium hydrogen carbonate, sodium hydroxide, triethylamine, diisopropylethylamine and the like.

The compound of the formula (I) wherein R³ is a halogen atom can be also prepared by reacting the compound of the formula (I) wherein R³ is a hydrogen atom, with a halogenating agent (bromine, N-bromosuccinimide, N-chlorosuccinimide, and so on) in an appropriate solvent.

The compound of the formula (I) wherein at least one of R¹ and R² are amino groups can be prepared by reacting the compound having a tert-butyloxycarbonylamino group on the corresponding position in the same method as Step 3 of Process A.

The compound of the formula (I) having a hydroxy group on the substituent of A or wherein R³ is a hydroxy group can be prepared by reacting the compound wherein the substituent on the corresponding position is methoxy group with boron tribromide or hydrobromic acid.

The compound of the formula (I) wherein R³ is an aryl group or an alkenyl group can be prepared by reacting the compound wherein R³ is a bromo group, and the corresponding aryl boronic acid, alkenyl boronic acid or an ester thereof, in the presence of a palladium catalyst such as tetrakis-triphenylphosphine or a base such as cesium carbonate and sodium carbonate, in a solvent such as toluene, dimethoxyethane, water or a mixture thereof, at about 20°C to about 120°C.

The compound of the formula (I) wherein R³ is a di (C₁₋₆ alkyl)amino group, 1-pyrrolidinyl group, 1-piperidinyl group or an aryl-C₁₋₆ alkylamino group can be prepared by reacting the compound wherein R³ is bromo group and each of various amine compounds in the same as Process A.

The compounds of the formula (I) prepared through the above-various processes can be isolated/purified by a conventional method such as chromatography, recrystallization, reprecipitation. The compound of the formula (I) may be provided in a free-base form or an acid addition salt form, depending on the type of the substituent in the structure, the selected starting material and the treating condition of the reaction, and can be transformed into the compound of the formula (I) according to a conventional method. The compound of the formula (I) having an enough basicity to form an acid addition salt can be converted into an acid addition salt thereof by treating with various acids according to a conventional method. In addition, the racemic compound of the formula (I) can be isolated/purified into an optically active form thereof according to a conventional method such as an optical resolution by chromatography with a optically active column, an optical resolution using an acid or a base as a synthetic chiral resolving agent, preferential crystallization, and diastereomer method. Likewise the intermediate compound of the formula (II) and the compound of the formula (VIIIb) can be also resolved into an optically active form. And using the optically active compound as a starting material, the optically active compound of the formula (I) can be prepared through the above method.

Hereinafter, pharmacological experiments and pharmacological activities of the representative compounds of the present invention are illustrated, but should not be construed to be limited thereto.

### Pharmacological test 1: Binding assay of central-type and mitochondrial (mitochondrial-type) benzodiazepine receptor

The binding assay of central-type benzodiazepine receptor (CBR) and the preparation of receptor membrane-preparation were carried out according to the partially modified method of Braestrup, C. et al. [see; Br. J. Psychiatry, 133, 249-260 (1978)] with a minor modification; the binding assay of mitochondrial benzodiazepine receptor (MBR) and the preparation of receptor membrane-preparation were carried out according to the method of Schoemaker, H [see; J. Pharmacol. Exp. Ther., 225, 61-69 (1983)] with a minor modification.

CBR and MBR membrane-preparations were prepared from forebrain (CBR) and kidney (MBR) of male Wistar strain rats according to the following procedure, respectively.

To the forebrain of rat, 20 volumes of ice-cold 0.32 M sucrose solution to wet-weight of the tissue (1:20, wet-weight/vol) was added and homogenized, and then the homogenate was centrifuged at 900xg for 10 minutes. The supernatant was centrifuged at 11,500xg for 20 minutes, a buffer I for the binding assay (50 mM Tris-hydrochloric acid buffer, pH 7.5) was added to the resultant precipitate, and the mixture was homogenized, and then it was centrifuged at 30,000×g for 10 minutes. The resulting precipitate was washed 3 times according to the same operation, suspended in the buffer I (1 g of wet-weight of tissue/10 ml) and used as a CBR membrane-preparation for the binding assay. The CBR membrane-preparation was stored at -80°C until use, and on the day of the assay, it was thawed and suspended in the buffer for use. On the other hand, kidney of rat was homogenized in 20 volumes of ice-cold buffer II for the binding assay to wet-weight of the tissue (1:20, wet-weight/vol) (50 mM sodium phosphate-potassium phosphate buffer solution containing 100 mM sodium chloride, pH 7.4), and then filtrated through 4 layers of gauze, and the filtrate was centrifuged at 40,000×g for 20 minutes. The resulting precipitate was suspended in the buffer II (1 g of wet-weight of tissue/50 ml) and used as a MBR membrane-preparation for the binding assay.

As a labeled ligand and a non-labeled ligand, [³H]flunitrazepam (final concentration: 1 nM) and diazepam (final concentration: 20 µM) were used respectively, for the CBR binding assay, and [³H]4'-chlorodiazepam (Ro5-4864, chemical name: 7-chloro-1,3-dihydro-1-methyl-5-(4-chlorophenyl)-2H-1,4-diazepin-2-one) (final concentration: 0.5 nM) and diazepam (final concentration: 50 µM) were used respectively, for the MBR binding assay. The binding assay of CBR was carried out by incubating at 0°C for 30 minutes, while that of MBR was at 0°C for 150 minutes.

The receptor binding assay was carried out in the following procedure. The test compound which the concentration was known, a tritium labeled ligand, a receptor membrane-preparation and the buffer I or II for the binding assay were added to a 96-well microplate in the total volume was 0.2 ml, and when the receptor membrane-preparation was added, the reaction started. After the incubation, the labeled ligand binding to the receptor was filtrated through a filter plate (UNI FILTER-96GF/B, PerkinElmer, the U.S.) using a cell harvester (PerkinElmer, the U.S.) to stop the reaction. The filter was immediately washed 6 times with 0.3ml of the ice-cold buffer [50 mM Tris-hydrochloric acid buffer (pH 7.7)]. After 30 µl of liquid scintillation cocktail (MICROSCINTI 20, PerkinElmer, the U.S.) was added to each well on the dried filter plate, the radioactivity was measured with a TopCount. The specific binding amount was obtained by subtracting the non-specific binding amount which was simultaneously measured in the presence of non-labeled ligand from the total binding amount. The concentration that the test compound could decrease the specific binding amount of the labeled ligand to 50% of the amount (IC₅₀ value) was analyzed using nonlinear least-squares method. The results of the binding assay of MBR are shown in Table 1. All the test compounds shown in Table 1 exhibited the IC₅₀ of more than 1000 nM in the CBR assay.

**Table 1**

| Example | IC₅₀ (nM) | Example | IC₅₀ (nM) | Example | IC₅₀ (nM) | Example | IC₅₀ (nM) |
|---|---|---|---|---|---|---|---|
| 1 | 1.98 | 36 | 1.28 | 75 | 2.77. | 129 | 5.56 |
| 4 | 3.90 | 37 | 3.23 | 76 | 3.04 | 130 | 4.04 |
| 5 | 2.28 | 38 | 1.88 | 77 | 3.15 | 132 | 10.1 |
| 6 | 2.92 | 39 | 1.71 | 78 | 2.53 | 133 | 3.95 |
| 7 | 123 | 40 | 6.05 | 79 | 1.76 | 134 | 33.8 |
| 8 | 2.05 | 41 | 2.41 | 83 | 9.33 | 135 | 13.5 |
| 10 | 1.14 | 42 | 14.3 | 84 | 5.58 | 136 | 67.0 |
| 11 | 1.84 | 48 | 77.0 | 85 | 3.69 | 139 | 7.15 |
| 12 | 1.37 | 49 | 3.98 | 90 | 6.54 | 140 | 3.84 |
| 13 | 2.08 | 50 | 92.6 | 91 | 2.31 | 141 | 12.3 |
| 14 | 0.823 | 51 | 21.2 | 93 | 5.81 | 143 | 8.69 |
| 15 | 1.46 | 54 | 6.50 | 94 | 16.7 | 145 | 31.0 |
| 16 | 1.17 | 55 | 55.1 | 98 | 44.3 | 146 | 4.91 |
| 17 | 0.638 | 57 | 2.03 | 105 | 80.7 | 147 | 3.80 |
| 18 | 1.47 | 58 | 4.31 | 107 | 28.5 | 148 | 44.7 |
| 21 | 1.23 | 59 | 12.9 | 108 | 17.7 | 149 | 5.35 |
| 22 | 19.3 | 60 | 1.54 | 110 | 30.9 | 150 | 5.36 |
| 23 | 0.949 | 61 | 1.42 | 111 | 13.3 | 151 | 3.59 |
| 24 | 0.851 | 62 | 12.2 | 118 | 4.22 | 152 | 7.29 |
| 25 | 6.73 | 63 | 2.51 | 119 | 3.98 | 153 | 1.13 |
| 26 | 0.851 | 67 | 1.56 | 122 | 26.8 | 154 | 5.00 |
| 27 | 1.66 | 68 | 1.67 | 123 | 1.45 | 155a | 4.36 |
| 28 | 5.72 | 69 | 4.51 | 124 | 2.39 | 155b | 8.69 |
| 30 | 1.29 | 70 | 2.45 | 125 | 1.33 | 156 | 2.76 |
| 32 | 0.727 | 72 | 0.659 | 126 | 2.69 | 158 | 28.5 |
| 33 | 1.99 | 73 | 3.35 | 127 | 1.97 | 159 | 61.5 |
| 35 | 1.19 | 74 | 2.44 | 128 | 3.91 | | |

The compound of the present invention shown in Table 1 can be strongly bound to MBR. Accordingly, it is apparent that the compounds of the present invention can be selectively and strongly bound to MBR, since the compounds exhibit the IC₅₀ of more than 1000 nM to CBR.

### Pharmacological test 2: Social interaction test (Study on antianxiety effect)

The test is a behavioral pharmacological test wherein social interaction time which would arise between 2 animals (mice, rats or other) in a test apparatus is considered to be as an anxious indicator [see; File, S.E., J. Neurosci. Methods, 2, 219-238 (1980)]. It is known that a bright and unfamiliar test apparatus which is an aversive condition for mice or rats may suppress a social interaction of the animals and such suppressed social interaction may be restored by an antianxiety drug such as benzodiazepines.

A glass beaker which was inverted onto a frosted glass plate was brightly illuminated with a light source (ca. 1200 lux in the apparatus), and it was used as a test apparatus. Two mice (male ddY, 22-32 g) that were housed in separate home cages were orally treated with a test compound and returned to their home cages. One hour after the oral administration, the two mice were then placed together in the test apparatus, and the amount of time spent in social interaction by the two mice during a 15-minute period was recorded. The social interaction was defined as grooming and sniffing of the partner, genital investigation of the partner, climbing over or crawling under the partner. Four to five pairs (8 to 10 mice) were used per a group.

An antianxiety effect of the test compounds was presented as a minimum effective dose which indicated statistically significant increase in social interaction time as compared with a vehicle control group (Dunnett's test, Significance level: 5%). The result is shown in Table 2.

**Table 2**

| Example | Minimum effective dose(mg/kg) |
|---|---|
| 1 | 0.001 |
| 23 | 0.01 |
| 24 | 0.001 |
| 40 | 1 |
| 41 | 0.01 |
| 57 | 0.1 |
| 63 | 0.01 |
| 129 | 1 |

In this test, it has been clarified that the compound of the present invention significantly increased the social interaction time with a dosage of no more than 1 mg/kg and thereby would exhibit an antianxiety effect.

### Pharmacological test 3: Forced swimming test (Study on antidepressant effect)

An animal (mouse or rat) which is forced to swim in an inescapable water tank show an immobile posture, thought to reflect a state of behavioral despair, after an initial period of vigorous swimming activity. The forced swimming test, a behavioral despair model, is frequently used for evaluating antidepressant effect since a lot of antidepressants reduce the duration of immobility induced by forced swimming. The antidepressant effect of the compound of the present invention was evaluated in the forced swimming test based on the method of Porsolt R. D. et al. [see; Eur. J. Pharmacol., 47, 379-391 (1978)] with a minor modification.

In the test, a group of five male Std-Wistar rats, weighing 110-150 g, which were selected after training session was used. The animals were subjected 4 times to training session which was carried out in an aquarium [23-25°C, transparent acrylic cylinder (i.d: 24.5 cm, height: 33 cm, depth of water: 15 cm) for 10 minutes once a day. And in the forth session, the animals which showed immobile time of over 180 seconds for the first 6 minutes after entering them into water were selected. On the fifth day, a 10 minute-training session was again done, and then the test was carried out on the seventh day.

The test compound (the compound of Example 40) was orally administered 3 times, i.e., 24 hours, 4 hours and 1 hour before the test. One hour after the final oral administration, the rats were put into the water and the immobile time observed during a 6-minute period was recorded. The effect shortening the immobile time in the test compound-treated group was evaluated using a Dunnett's multiple comparison test as compared with the vehicle control group.

The compound of Example 40 significantly shortened the immobile time by 28% at the dose of 1 mg/kg (p<0.01).

From the above results of the pharmacological tests, apparently the compounds of the present invention exhibit a selective and strong affinity for MBR in the in vitro tests and additionally exhibit a potent antianxiety effect and antidepressant effect in the animal tests. Therefore the compounds of the present invention will be useful as a medicament for treating/preventing anxiety disorders (panic disorder, generalized anxiety disorder, social-anxiety disorder, obsessive-compulsive disorder, posttraumatic stress disorder and other), depressions /mood disorder, epilepsy, dementia (Alzheimer's disease, cerebrovascular dementia and other), anxiety and depression, sleep disorder, nervous disease (Huntington's disease, multiple sclerosis, peripheral nerve disease and other), stress-related gastrointestinal disorders (stomach and duodenal ulcer, irritable bowel syndrome and other), inflammatory disease (rheumatoid arthritis and other), and cancer.

The route for the administration is not specifically restricted and may be administered via oral or parenteral such as endorectal and percutaneous. The dosage varies depending upon administering pattern, conditions and age of patient, purpose of treatment (prevention or treatment) and so on, generally 0.01-50 mg/kg/day, preferably 0.03-10 mg/kg/day, more preferably 0.1-4 mg/kg/day.

The compound of the present invention is usually administered as a pharmaceutical composition prepared by mixing the compound with pharmaceutical carrier(s). The carrier(s) used for pharmaceutical composition are materials that are conventional and inert to the compound of the present invention. For example, lactose, inositol, glucose, mannitol, dextran, cyclodextrin, sorbitol, starch, partly pregelatinized starch, sugar, magnesium aluminometasilicate, synthetic aluminum silicate, crystalline cellulose, sodium carboxymethyl cellulose, hydroxypropyl starch, calcium carboxymethyl cellulose, ionexchange resin, methyl cellulose, gelatin, gum arabic, hydroxypropyl cellulose, low subtituted hydroxypropyl cellulose, hydroxypropylmethyl cellulose, polyvinyl pyrrolidone, polyvinyl alcohol, alginic acid, sodium alginate, light anhydrous silicic acid, magnesium stearate, talc, carboxyvinyl polymer, titanium oxide, sorbitan fatty acid esters, sodium lauryl sulfate, glycerin, glycerol esters of fatty acids, purified lanolin, glycerogelatin, polysorbate, macrogol, vegetable oil, wax, propyleneglycol, water, ethanol, polyoxyethylene hydrogenated castor oil (HCO), sodium chloride, sodium hydroxide, hydrochloric acid, disodium hydrogen phosphate, sodium dihydrogen phosphate, citric acid, glutamic acid, benzyl alcohol, methyl p-oxybenzoate, ethyl p-oxybenzoate, white vaseline, plastibase, white beeswax, macrogol are exemplified.

The drug formulations of the present invention include tablets, capsules, granules, powders, syrups, suspensions; suppositories, injections, ointments, cataplasms and so on. These drug formulations can be prepared according to a conventional method. The liquid preparations may be prepared by solving or suspending the drug in water or other appropriate solvents just when used. The tablets and granules may be coated by a well-known method. The injection preparations can be prepared by dissolving the compound of the present invention in water; and when necessary, an isotonic agent or a solubilizer may be used to dissolve it, or a pH adjusting agent, a buffering agent or a preservative may be also added thereto.

These drug formulations may contain the compound of the present invention in an amount of more 0.01% by weight, preferably 0.1-70 % by weight. These drug formulations may optionally contain other therapeutically effective materials.

Further, properly isotope-labeled agents of the invention (i.e. compounds of formula (I)) exhibit valuable properties as histopathological labeling agents, imaging agents and/or biomarkers, hereinafter "markers", for the selective labeling of the MBR. More particularly the agents of the invention are useful as markers for labeling the MBRs in vitro or in vivo.

In particular, compounds of the invention which are properly isotopically labeled are useful as ligands to image MBR in vivo or in vitro studies. Suitable radionuclides that may be incorporated in the agents of invention include: ³H, ¹¹C, ¹³N ¹⁵O, ¹⁸F, ¹²³I, ¹²⁵I, ¹³¹I, ⁷⁵Br, ⁷⁶Br, ⁷⁷Br, ⁸²Br, ^{99m}Tc and ²¹¹At. The choice of radionuclide to be incorporated into the compounds of formula (I) will depend on the specific analytical or pharmaceutical application. Therefore, for in vitro labeling of MBRs and for competition assays the compounds that incorporate ³H, ¹²⁵I or ⁷⁷Br would be preferred. For diagnostic and investigating imaging agents (PET or SPECT) the compounds that incorporate a radionuclide selected from ¹¹C, ¹⁸F, ¹²³I or ⁷⁶Br are preferred.

The agents of the invention are therefore useful, for instance, for determining the levels of receptor occupancy of a drug acting at the MBR, or diagnostic purposes for diseases resulting from an imbalance or dysfunction of MBRs, and for monitoring the effectiveness of pharmacotherapies of such diseases.

In accordance with the above, the present invention provides an agent of the invention for use as a marker for neuroimaging.

In a further aspect, the present invention provides a composition for labeling brain and peripheral nervous system structures involving MBRs in vivo and in vitro comprising an agent of the invention.

In still a further aspect, the present invention provides a method for labeling brain and peripheral nervous system structures involving MBRs in vitro or in vivo, which comprises contacting brain tissue with an agent of the invention.

The method of the invention may comprise a further step aimed at determining whether the agent of the invention labeled the target structure. Said further step may be effected by observing the target structure using positron emission tomography (PET) or single photon emission computed tomography (SPECT), or any device allowing detection of radioactive radiations.

### BEST MODE FOR CARRYING OUT THE INVENTION

Hereinafter, the present invention is further illustrated by Reference Examples and Examples, but should not be construed to be limited thereto. The identification of the compounds was carried out using elemental analysis, mass spectrum, IR spectrum, NMR spectrum on so on. The optical purity of the optically active compounds was measured by HPLC.

The following abbreviate symbols are optionally used in the tables in Reference Examples and Examples herein to simplify the chemical names.
EtOH: ethanol, AcOEt: ethyl acetate, i-PrOH: isopropanol,
Et₂O: diethyl ether, (i-Pr)₂O: diisopropyl ether,
Boc: tert-butoxycarbonyl, Ac: acetyl, Ph: phenyl.

### Reference Example 1

### Preparation of 1-(tert-butoxycarbonyl)indoline-2-carboxylic acid:

To 80 g of indoline-2-carboxylic acid were added 400 ml of 1,4-dioxane and 980 ml of 0.5 mol/l aqueous sodium hydroxide, followed by slowly adding dropwise a mixture of 118 g of di-tert-butyl dicarbonate and 150 ml of 1,4-dioxane at 0°C. The mixture was stirred for 14 hours at room temperature and then 300 ml of hexane was added thereto. The aqueous layer was allowed to be acidic with 10% aqueous citric acid, and then the reaction mixture was extracted with ethyl acetate and the extract was washed with brine. The extract was dried over anhydrous sodium sulfate and filtrated. The filtrate was concentrated in vacuo. The residue was recrystallized from hexane-ethyl acetate to give 86 g of the desired compound.
Melting point: 127-128°C

### Reference Example 2

### Preparation of (R)-1-(tert-butoxycarbonyl)indoline-2-carboxylic acid:

(R)-Indoline-2-carboxylic acid was reacted and treated as a starting compound in the similar manner as Reference Example 1 to give the desired compound.
Melting point: 126-129°C

### Reference Example 3

### Preparation of (S)-1-(tert-butoxycarbonyl)indoline-2-carboxylic acid:

(S)-Indoline-2-carboxylic acid was reacted and treated as a starting compound in the similar manner as Reference Example 1 to give the desired compound.
Melting point: 131-133°C

### Reference Example 4

### Preparation of tert-butyl 2-[(cis-3,5-dimethylpiperidin-1-yl)carbonyl]indoline=1-carboxylate:

85 g of 1-(tert-Butoxycarbonyl)indoline-2-carboxylic acid and 48.3 g of cis-3,5-dimethylpiperidine hydrochloride were dissolved in 600 ml of tetrahydrofuran, and thereto was added 47.5 g of 1-hydroxybenzotriazole. The mixture was cooled to 0°C, and then thereto was slowly added dropwise 54.6 g of 1-ethyl-3-(3-dimethylaminopropyl)-carbodiimide and the mixture was stirred for 15 hours at room temperature. After evaporation of tetrahydrofuran, the residue was dissolved in ethyl acetate and the solution was washed with 10% aqueous citric acid, water, saturated aqueous sodium hydrogen carbonate and brine. The solution was dried over anhydrous sodium sulfate, and filtrated. The filtrate was concentrated and to the residue was added diisopropyl ether. The mixture was stirred and then was filtered and the collected crystal was dried to give 112 g of the desired compound.
Melting point: 148-149°C

### Reference Examples 5-11

1-(tert-Butoxycarbonyl)indoline-2-carboxylic acid was reacted and treated with various cyclic amines in the similar manner as Reference Example 4 to prepare the following compounds.

### (Reference Example 5)

### tert-Butyl 2-[(1-piperidinyl)carbonyl]indoline-1-carboxylate (Melting point: 131-133°C)

### (Reference Example 6)

### tert-Butyl (R)-2-[(cis-3,5-dimethylpiperidin-1-yl)-carbonyl]indoline-1-carboxylate (Melting point: 133-134°C)

### (Reference Example 7)

### tert-Butyl (S)-2-(cis-3,5-dimethylpiperidin-1-yl)-carbonyl]indoline-1-carboxylate (Melting point: 134-135°C)

### (Reference Example 8)

### tert-Butyl (S)-2-[(3,5-dimethylpiperidin-1-yl)carbonyl]-indoline-1-carboxylate

### (Reference Example 9)

### tert-Butyl 2-[(3-methylpiperidin-1-yl)carbonyl]indoline-1-carboxylate (Melting point: 133-135°C)

### (Reference Example 10)

### tert-Butyl (S)-2-[[(R)-3-methylpiperidin-1-yl]carbonyl]-indoline-1-carboxylate (Melting point: 127-130°C)

### (Reference Example 11)

### tert-Butyl (S)-2-[[(S)-3-methylpiperidin-1-yl]carbonyl]-indoline-1-carboxylate (Melting point: 116-119°C)

### Reference Example 12

### Preparation of 2-[(cis-3,5-dimethylpiperidin-1-yl)-carbonyl]indoline:

110 g of tert-Butyl 2-[(cis-3,5-dimethylpiperidin-1-yl)carbonyl]indoline-1-carboxylate was dissolved in 200 ml of dichloromethane, and thereto was added 200 ml of trifluoroacetic acid and the mixture was stirred for 2 hours at room temperature. The solvent was evaporated and then to the residue was added ethyl acetate, and the mixture was allowed to be neutral with aqueous sodium hydroxide. The organic layer was washed with brine, and then dried over anhydrous sodium sulfate and filtered. The filtrate was concentrated in vacuo. The residue was recrystallized from diisopropyl ether to give 75 g of the desired compound.
Melting point: 131-133°C

### Reference Examples 13-19

The compounds obtained in Reference Examples 5-11 were reacted and treated in the similar manner as Reference Example 12, and the products were optionally purified by silica gel column chromatography (hexane:ethyl acetate = 5:1) to prepare the following compounds.

### (Reference Example 13)

### 2-[(1-Piperidinyl)carbonyl]indoline (Melting point: 121-123°C)

### (Reference Example 14)

### (R)-2-[(cis-3, 5-Dimethylpiperidin-1-yl)carbonyl]indoline (Melting point: 93-95°C)

### (Reference Example 15)

### (S)-2-[(cis-3,5-Dimethylpiperidin-1-yl)carbonyl]indoline (Melting point: 100-102°C)

### (Reference Example 16)

### (S)-2-[(trans-3,5-Dimethylpiperidin-1-yl)carbonyl]indoline (Reference Example 17)

### 2-[(3-Methylpiperidin-1-yl)carbonyl]indoline (Melting point: 111-113°C)

### (Reference Example 18)

### (S)-2-[[(R)-3-Methylpiperidin-1-yl]carbonyl]indoline (Melting point: 92-94°C)

### (Reference Example 19)

### (S)-2-[[(S)-3-Methylpiperidin-l-yl]carbonyl]indoline. (Melting point: 119-121°C)

### Reference Example 20

### Preparation of 1-(2-pyrimidinyl)indoline-2-carboxylic acid:

To 15 g of 2-[(1-piperidinyl)carbonyl]-1-(2-pyrimidinyl)indoline was added 100 ml of 2 mol/l hydrochloric acid, and the mixture was heated to reflux for 3 hours. The mixture was cooled to 0°C, then allowed to be alkaline by adding aqueous sodium hydroxide and washed with diethyl ether. To the aqueous layer was added concentrated hydrochloric acid, and the precipitated crystal was filtered, washed with water and dried by heating to give 9.0 g of the desired compound.
Melting point: 249-250°C

### Reference Example 21

### Preparation of 1-(tert-butoxycarbonyl)-4-methoxyindoline-2-carboxylic acid:

Methyl 4-methoxyindoline-2-carboxylate was reacted and treated as a starting compound in the similar manner as Reference Example 1 to give the desired compound.
(Melting point: 148-150°C)

### Reference Example 22

### Preparation of 1-(tert-butoxycarbonyl)-6-methoxyindoline-2-carboxylic acid:

Methyl 6-methoxyindoline-2-carboxylate was reacted and treated as a starting compound in the similar manner as Reference Example 1 to give the desired compound.'
(Melting point: 154-156°C)

### Reference Examples 23-26

The compounds obtained in Reference Example 2, Reference Example 21 or Reference Example 22 were reacted and treated with various cyclic amines in the similar manner as Reference Example 4 to prepare the following compounds.

### (Reference Example 23)

### tert-Butyl (R)-2-[[(S)-3-methylpiperidin-l-yl]carbonyl]-indoline-1-carboxylate (Melting point: 132-136°C)

### (Reference Example 24)

### tert-Butyl (R)-2-[[(R)-3-methylpiperidin-1-yl]carbonyl]-indoline-1-carboxylate (Melting point: 118-121°C)

### (Reference Example 25)

### tert-Butyl 4-methoxy-2-[[(S)-3-methylpiperidin-1-yl]-carbonyl]indoline-1-carboxylate

(Melting point:156-158°C)

### (Reference Example 26)

### tert-Butyl 6-methoxy-2-[[(S)-3-methylpiperidin-1-yl]-carbonyl]indoline-1-carboxylate

(Melting point: 146-148°C)

### Reference Examples 27-30

The compounds obtained in Reference Examples 23-26 were reacted and treated in the similar manner as Reference Example 12, and the products were optionally purified by silica gel column chromatography (hexane:ethyl acetate = 5:1) to prepare the following compounds.

### (Reference Example 27)

### (R)-2-[[(S)-3-Methylpiperidin-1-yl]carbonyl]indoline

(Melting point: 94-95°C)

### (Reference Example 28)

### (R) -2- [[(R)-3-Methylpiperidin-1-yl] carbonyl] indoline

(Melting point: 120-122°C)

### (Reference Example 29)

### 4-Methoxy-2-[[(S)-3-methylpiperidin-1-yl]carbonyl]indoline

### (Reference Example 30)

### 6-Methoxy-2-[[(S)-3-methylpiperidin-1-yl]carbonyl]indoline

(Melting point: 92-94°C).

### Reference Example 31

### Preparation of 5-methoxy-2-[[(S)-3-methylpiperidin-1-yl]-carbonyl]indole:

50 g of 5-Methoxyindole-2-carboxylic acid and 31.1 g of (S)-3-methylpiperidine were dissolved in 500 ml of dimethylformamide and the mixture was cooled to 0°C, and then thereto was slowly added 65.3 g of 1-ethyl-3-(3-dimethylaminopropyl)carbodiimide hydrochloride and the mixture was stirred for 3 hours at room temperature. To the reaction mixture was added 500 ml of water, and the precipitated crystal was filtrated. The crystal was dried to give 60 g of the desired compound.
(Melting point: 189-192°C)

### Reference Examples 32-33

5-Fluoroindole-2-carboxylic acid or 5-bromo-3-methyl indole-2-carboxylic acid was reacted and treated as a starting compound in the similar manner as Reference Example 31 to give the following compounds.

### (Reference Example 32)

### 5-Fluoro-2-[[(S)-3-methylpiperidin-1-yl]carbonyl]indole

(Melting point: 162-164°C)

### (Reference Example 33)

### 5-Bromo-3-methyl-2-[[(S)-3-methylpiperidin-1-yl]carbonyl]-indole

(Melting point: 190-192°C)

### Reference Example 34

### Preparation of 3-methyl-2-[[(S)-3-methylpiperidin-1-yl]-carbonyl]indole:

12.7 g of 5-Bromo-3-methyl-2-[[(S)-3-methylpiperidin-1-yl]carbonyl]indole was suspended in 200 ml of ethanol, and thereto were added 19.4 ml of 1 mol/l aqueous sodium hydroxide and 1.0 g of 10% palladium carbon at 0°C. The mixture was stirred for 4 hours at room temperature under hydrogen atmosphere. The reaction mixture was filtrated through Celite and then the filtrate was concentrated in vacuo and to the residue was added ethyl acetate. The solution was washed with water and brine. The solution was dried over anhydrous sodium sulfate and filtered. The filtrate was concentrated in vacuo. The product was recrystallized from ethanol to give 7.8 g of the desired compound.
(Melting point: 198-200°C)

### Reference Example 35

### Preparation of 5-methoxy-2-[[(S)-3-methylpiperidin-1-yl]-carbonyl]indoline:

20 g of 5-Methoxy-2-[[(S)-3-methylpiperidin-1-yl]-carbonyl]indole was suspended in 400 ml of methanol, and thereto was added 12.5 g of magnesium at 0°C. The mixture was stirred for 4 hours at room temperature under nitrogen atmosphere. The reaction mixture was added at 0°C to 400 ml of 1 mol/l hydrochloric acid, and thereto was added water after evaporating methanol, and the mixture was allowed to be neutral with aqueous sodium hydroxide. The mixture was extracted with ethyl acetate, and then the extract was washed with water and brine. The extract was dried over anhydrous sodium sulfate and filtered. The filtrate was concentrated in vacuo. The residue was purified by silica gel column chromatography eluted by hexane/ethyl acetate (3:1) to give 11 g of the desired compound as an oil.

### Reference Examples 36-37

5-Fluoro-2-[[(S)-3-methylpiperidin-1-yl]carbonyl]-indole or 3-methyl-2-[[(S)-3-methylpiperidin-1-yl]-carbonyl]indole was reacted and treated as a starting compound in the similar manner as Reference Example 35 to prepare the following compounds.

### (Reference Example 36)

### 5-Fluoro-2-[[(S)-3-methylpiperidin-1-yl]carbonyl]indoline

(Melting point: 104-107°C)

### (Reference Example 37)

### 3-Methyl-2-[[(S)-3-methylpiperidin-1-yl]carbonyl]indoline

(Melting point: 115-117°C)

### Reference Example 38

### Preparation of (S)-1-(6-chloropyridazin-3-yl)indoline-2-carboxylic acid:

14.1 g of Sodium tert-butoxide was suspended in 100 ml of tetrahydrofuran, and thereto was added a suspension of 10 g of (S)-indoline-2-carboxylic acid in 50 ml of tetrahydrofuran at room temperature under nitrogen atmosphere, followed by adding dropwise a solution of 9.6 g of 3,6-dichloropyridazine in 40 ml of THF. The mixture was stirred for 30 minutes at room temperature, and thereto was added 500 ml of water under ice-cooling. Then, the mixture was allowed to be acidic (pH 3-4) by adding 1 mol/l hydrochloric acid. The precipitated crystal was filtrated, washed with water and dried to give 13.7 g of the desired compound.
Melting point: 211-212°C

### Reference Example 39

### Preparation of (R)-1-(6-chloropyridazin-3-yl)indoline-2-carboxylic acid:

(R)-Indoline-2-carboxylic acid was reacted and treated as a starting compound in the similar manner as Reference Example 38 to give the desired compound.
Melting point: 213-214°C

### Reference Example 40

### Preparation of (S)-1-(1-methyl-6-oxo-1,6-dihydropyridazin-3-yl)indoline-2-carboxylic acid:

11 g of (S)-1-(6-Chloropyridazin-3-yl)indoline-2-carboxylic acid was suspended in 200 ml of 1,4-dioxane, and thereto**·**was added dropwise 25.2 g of dimethyl sulfate under ice-cooling. The mixture was stirred for 6 hours at room temperature, and then stirred for 2 hours at 50°C. The mixture was cooled to room temperature, and the precipitate was filtrated and washed with diethyl ether to give a yellow solid. The yellow solid was dissolved in 120 ml of water, and thereto was added dropwise 120 ml of 1 mol/l aqueous, sodium hydroxide under ice-cooling. The mixture was warmed to room temperature and stirred for 2 hours, and then allowed to be acidic (pH 1-2) by adding dropwise hydrochloric acid under ice-cooling. The precipitated crystal was filtrated, washed with water and dried to give 9.9 g of the desired compound.
Melting point: 245-247°C

### Reference Example 41

### Preparation of (R)-1-(1-methyl-6-oxo-1,6-dihydropyridazin-3-yl)indoline-2-carboxylic acid:

(R)-1(-6-Chloropyridazin-3-yl) indoline-2-carboxylic acid was reacted and treated as a starting compound in the similar manner as Reference Example 40 to give the desired compound.
Melting point: 240-243°C

### Reference Example 42

### Preparation of methyl (S)-1-(6-chloropyridazin-3-yl)-indoline-2-carboxylate:

60 g of (S)-1-(6-Chloropyridazin-3-yl)indoline-2-carboxylic acid was dissolved in 600 ml of dimethylformamide, and thereto was added 58.3 ml of dimethylformamide dimethyl acetal. The mixture was stirred for 15 hours at room temperature. After completion of the reaction, the reaction mixture was poured into 2.4 L of ice water and then the precipitate was filtrated. The resulting precipitate was washed with water and dried to give 47.9 g of the desired compound.
(Melting point: 133-134°C)

### Reference Example 43

### Preparation of (S)-1-(1-methyl-6-oxo-1,6-dihydropyridazin-3-yl)indoline-2-carboxylic acid:

20 g of the compound obtained in Reference Example 42 and 60.8 g of trimethylsulfoxonium iodide were added to 100 ml of N-methylpyrrolidone, and the mixture was stirred for 2 hours at 95°C. After cooling the reaction mixture to room temperature, the solution was added dropwise to ice-cooled 500 ml of 1 mol/l aqueous sodium hydroxide and then stirred for 10 minutes at room temperature. The mixture was ice-cooled again and allowed to be acidic (pH 4) by adding 2 mol/l hydrochloric acid, and the precipitated crystal was filtrated. The precipitate was washed with water and ethyl acetate, and then dried to give 15.3 g of the desired compound.

### Reference Example 44

### Preparation of methyl (S)-1-(6-oxo-1,6-dihydropyridazin-3-yl)indoline-2-carboxylate:

42 g of Methyl (S)-1-(6-chloropyridazin-3-yl)indoline-2-carboxylate was dissolved in 400 ml of acetic acid, and the mixture was heated to reflux for 15 hours. After completion of the reaction, the reaction mixture was poured into 1.6 L of ice water and then the precipitate was filtrated. The precipitate was washed with water and dried to give 34.5 g of the desired compound.
(Melting point: 220°C (dec.))

### Reference Example 45

### Preparation of methyl (S)-1-(1-benzyl-6-oxo-1,6-dihydro-pyridazin-3-yl)indoline-2-carboxylate:

0.44 g of Methyl (S)-1-(6-oxo-1,6-dihydropyridazin-3-yl) indoline-2-carboxylate was dissolved in 10 ml of dimethylformamide, and thereto were added 0.33 g of benzyl bromide and 0.67 g of potassium carbonate. The mixture was stirred for 3 hours at room temperature. After completion of the reaction, the reaction mixture was poured into 50 ml of ice water and then the precipitate was filtrated. The precipitate was washed with water and dried to give 0.59 g of the desired compound.
(Melting point: 163-165°C)

### Reference Examples 46-50

Methyl (S)-1-(6-oxo-1,6-dihydropyridazin-3-yl)-indoline-2-carboxylate was reacted and treated with various halogenated compounds in the similar manner as Reference

### Example 45 to prepare the following compounds.

### (Reference Example 46)

### Methyl (S)-1-(1-cyclopentyl-6-oxo-1,6-dihydropyridazin-3-yl)indoline-2-carboxylate

### (Reference Example 47)

### Methyl (S)-1-(1-cyclobutylmethyl-6-oxo-1,6-dihydro-pyridazin-3-yl)indoline-2-carboxylate

### (Reference Example 48)

### Methyl (S)-1-[6-oxo-1-(2-oxo-2-phenylethyl)-1,6-dihydro-pyridazin-3-yl]indoline-2-carboxylate

### (Reference Example 49)

### Methyl (S)-1-[1-(2-fluoroethyl)-6-oxo-1,6-dihydropyridazin-3-yl]indoline-2-carboxylate

(Melting point: 168-170°C)

### (Reference Example 50)

### Methyl (S)-1-[1-(2,2-difluoroethyl)-6-oxo-1,6-dihydro-pyridazin-3-yl]indoline-2-carboxylate

### Reference Example 51

### Preparation of (S)-1-(1-benzyl-6-oxo-1,6-dihydropyridazin-3-yl)indoline-2-carboxylic acid:

0.59 g of Methyl (S)-1-(1-benzyl-6-oxo-1,6-dihydro-pyridazin-3-yl)indoline-2-carboxylate was dissolved in 8 ml of tetrahydrofuran, and thereto were added 4 ml of water and 0.1 g of lithium hydroxide monohydrate. The mixture was stirred for 3 hours at room temperature. The reaction mixture was adjusted to pH 1 by adding diluted hydrochloric acid, and then the precipitate was washed with water and dried to give 0.32 g of the desired compound.
(Melting point: 245°C (dec.))

### Reference Examples 52-56

The compounds obtained in Reference Examples 46-50 were reacted and treated in the similar manner as Reference Example 51 to give the following compounds.

### (Reference Example 52)

### (S)-1-(1-Cyclopentyl-6-oxo-1,6-dihydropyridazin-3-yl)-indoline-2-carboxylic acid

### (Reference Example 53)

### (S)-1-(1-Cyclobutylmethyl-6-oxo-1,6-dihydropyridazin-3-yl)indoline-2-carboxylic acid

(Melting point: 243°C (dec.))

### (Reference Example 54)

### (S)-1-[6-Oxo-1-(2-oxo-2-phenylethyl)-1,6-dihydropyridazin-3-yl]indoline-2-carboxylic acid

(Melting point: 243°C (dec.))

### (Reference Example 55)

### (S)-1-[1-(2-Fluoroethyl)-6-oxo-1,6-dihydropyridazin-3-yl]indoline-2-carboxylic acid

(Melting point: 179°C (dec.))

### (Reference Example 56)

### (S)-1-[1-(2,2-Difluoroethyl)-6-oxo-1,6-dihydropyridazin-3-yl]indoline-2-carboxylic acid

(Melting point: 207°C (dec.))

### Reference Example 57

### Preparation of methyl 1-(1-methyl-6-oxo-1,6-dihydro-pyridazin-3-yl)-2-methylindoline-2-carboxylate:

To a solution of 0.73 ml of 1,1,1,3,3,3-hexamethyldisilazane in 30 ml of tetrahydrofuran was added 2.18 ml of a solution of 1.6 mol/l n-butyllithium in hexane under nitrogen atmosphere at -78°C, and the mixture was stirred for 30 minutes at room temperature. Then, a solution of 0.50 g of methyl (S)-1-(1-methyl-6-oxo-1,6-dihydro-pyridazin-3-yl)indoline-2-carboxylate in 15 ml of tetrahydrofuran was added dropwise at -78°C thereto, and the mixture was stirred for 1 hour at the same temperature. Then, 0.22 ml of methyl iodide was added at -78°C thereto, and the mixture was stirred for 1 hour at the same temperature, and then warmed to 0°C and stirred for another 1 hour. 13 ml of 2 mol/l hydrochloric acid was added dropwise at 0°C thereto, and the mixture was extracted with ethyl acetate and the extract was washed with brine. Then, the extract was dried over magnesium sulfate and filtrated. The filtrate was concentrated. The residue was purified by silica gel column chromatography eluted by chloroform/methanol (50:1) to give 0.50 g of the desired compound as amorphous.
¹H NMR (CDCl₃) δ: 7.48 (1H, d, J=10.0Hz), 7.2-7.1 (2H, m), 6.95 (1H, d, J=10.0Hz), 6.9-6.8 (2H, m), 3.87 (3H, s), 3.84 (3H, s), 3.43 (1H, d, J=16.0Hz), 3.13 (1H, d, J=16.0Hz), 1.74 (3H, s)

### Reference Example 58

### Preparation of 1-(1-methyl-6-oxo-1,6-dihydropyridazin-3-yl)-2-methylindoline-2-carboxylic acid:

To a solution of 0.50 g of the compound obtained in Reference Example 57 in 30 ml of tetrahydrofuran were added 5 ml of water and 0.21 g of lithium hydroxide monohydrate, and the mixture was heated to reflux for 30 minutes. 2.5 ml of 2 mol/l Hydrochloric acid was added dropwise at 0°C thereto, and the mixture was concentrated in vacuo and the residue was purified by silica gel column chromatography eluted by chloroform/methanol (10:1) to give 0.34 g of the desired compound as amorphous.
¹H NMR (CDCl₃) δ: 7.50 (1H, d, J=10.0Hz), 7.2-7.0 (3H, m), 7.0-6.8 (2H, m), 3.67 (3H, s), 3.58 (1H, d, J=16.0Hz), 3.14 (1H, d, J=16.0Hz), 1.74 (3H, s)

### Example 1

### Preparation of 2-[(cis-3,5-dimethylpiperidin-1-yl)-carbonyl]-1-(2-pyrimidinyl)indoline:

1.0 g of 2-[(cis-3,5-Dimethylpiperidin-1-yl)carbonyl]-indoline, 0.44 g of 2-chloropyrimidine, 0.18 g of tris-(dibenzylideneacetone)dipalladium, 0.48 g of sodium tert-butoxide and 31 mg of tri-tert-butylphosphine were dissolved in dry toluene under nitrogen atmosphere, and the mixture was stirred for 2 hours at room temperature. The reaction mixture was filtrated through Celite and the filtrate was concentrated in vacuo, and the residue was purified by silica gel column chromatography eluted by hexane/ethyl acetate (3:1) and recrystallized from isopropanol to give 0.40 g of the desired compound.
Melting point: 194-196°C

### Example 2

### Preparation of (S)-2-[(cis-3,5-dimethylpiperidin-1-yl)-carbonyl]-1-(1,3-thiazol-2-yl)indoline:

1.0 g of (S)-2-[(cis-3,5-Dimethylpiperidin-1-yl)-carbonyl]indoline and 0.63 g of 2-bromothiazole were dissolved in 10 ml of toluene under nitrogen atmosphere, and thereto was added 0.48 g of sodium tert-butoxide at 0°C. The mixture was stirred for 2 hours at room temperature, and then thereto was added ethyl acetate and the mixture was washed with water and brine. The mixture was dried over anhydrous sodium sulfate and filtrated. The filtrate was concentrated in vacuo. The residue was purified by silica gel column chromatography eluted by hexane/ethyl acetate (3:1) and recrystallized from isopropanol to give 0.28 g of the desired compound.
Melting point: 172-174°C

### Example 3

### Preparation of (S)-2-[(cis-3, 5-dimethylpiperidin-1-yl)-carbonyl]-1-(3-nitropyridin-2-yl)indoline:

0.7 g of (S)-2-[(cis-3, 5-Dimethylpiperidin-1-yl)-carbonyl]indoline, 0.43 g of 2-chloro-3-nitropyridine and 0.27 g of triethylamine were dissolved in 10 ml of toluene, and the mixture was heated to reflux for 2 hours. The reaction mixture was concentrated in vacuo, and then the residue was purified by silica gel column chromatography eluted by hexane/ethyl acetate (3:1) to give 0.7 g of the amorphous desired compound.
¹H NMR (CDCl₃) δ: 8.45-8.33 (2H, m), 7.19 (1H, d, J=8.0Hz), 7.09 (1H, t, J=8.0Hz),- 6:97-6.90 (2H, m), 6.51 (1H, d, J=8.0Hz), 5.70-5.67 (1H, m), 4.50 (1H; d, J=12.9Hz), 3.91 (1H, d, J=13.2Hz), 3.61-3.52 (1H, m), 3.13 (1H, dd, J=15.2, 5.7Hz), 2.75-2.61 (1H, m), 2.10-1.45 (4H, m), 1.14-0.83 (7H, m)

### Examples 4-42

The compounds obtained in Reference Examples 12-1-9 were reacted and treated with various halogenated heteroaromatic compounds in the similar manner as Example 1 (Preparation I), Example 2 (Preparation II) or Example 3 (Preparation III) to give the compounds of Examples 4-42 in Table 3, Table 4 and Table 5. Both the optical purities of the compounds of Example 24 and Example 42 were more than 99.5% ee.

**Table 3**

| | | | | | | | |
|---|---|---|---|---|---|---|---|
| 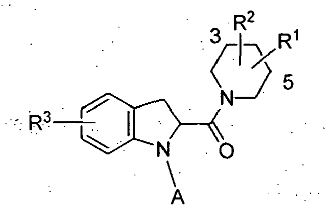 | | | | | | | |

| Ex. | A | R¹ | R² | R³ | M.P. | Recrystallizing Solvent | Preparation |
|---|---|---|---|---|---|---|---|
| 1 | 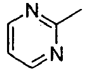 | cis-3-CH₃ | cis-5-CH₃ | H | 194-196 | i-PrOH | I |
| 4 | 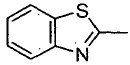 | cis-3-CH₃ | cis-5-CH₃ | H | 195-197 | AcOEt | I |
| 5 | 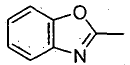 | cis-3-CH₃ | cis-5-CH₃ | H | 199-201 | AcOEt | I |
| 6 | 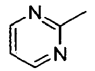 | 3-CH₃ | H | H | 156-158 | AcOEt | I |
| 7 | 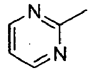 | H | H | H | 149-151 | AcOEt | I |
| 8 | 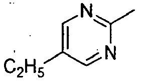 | cis-3-CH₃ | cis-5-CH₃ | H | 147-149 | i-PrOH | I |
| 9 | 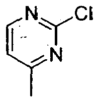 | cis-3-CH₃ | cis-5-CH₃ | H | 214-216 | i-PrOH | I |
| 10 | 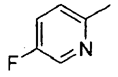 | cis-3-CH₃ | cis-5-CH₃ | H | 139-141 | (i-Pr)₂O | I |
| 11 | 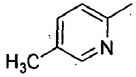 | cis-3-CH₃ | cis-5-CH₃ | H | 137-139 | (i-Pr)₂O | I |
| 67 | 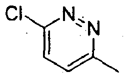 | (*S*)-3-CH₃ | H | 5-F | 164-168 | i-PrOH | II |
| 68 | 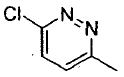 | (*S*)-3-CH₃ | H | 4-OCH₃ | 223-226 | ETOH EtOH | II |
| 69 | 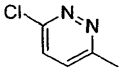 | (*S*)-3-CH₃ | H | 5-OCH₃ | 136-139 | AcOEt | II |
| 70 | 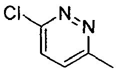 | (*S*)-3-CH₃ | H | 6-OCH₃ | 184-186 | ETOH EtOH | II |

**Table 4**

| | | | | | | |
|---|---|---|---|---|---|---|
| 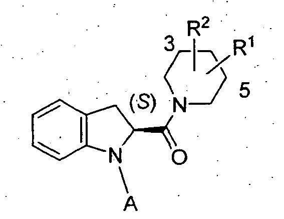 | | | | | | |

| Ex. | A | R¹ | R² | M.P. (°C) | Recrystallizing solvent | Preparation |
|---|---|---|---|---|---|---|
| 2 | 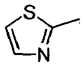 | cis-3-CH₃ | cis-5-CH₃ | 172-174 | i-PrOH | II |
| 3 | 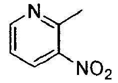 | cis-3-CH₃ | cis-5-CH₃ | amorphous | | III |
| 12 | 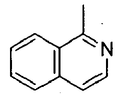 | (*S*)-3-CH₃ | H | 134-136 | AcOEt + (i-Pr)₂O | I |
| 13 | 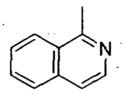 | cis-3-CH₃ | cis-5-CH₃ | 173-175 | AcOEt | I |
| 14 | 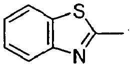 | (*S*)-3-CH₃ | H | 165-166 | AcOEt | I |
| 15 | 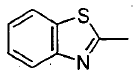 | cis-3-CH₃ | Cis-5-CH₃ | 170-173 | AcOEt + (i-Pr) ₂O | I |
| 16 | 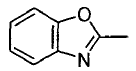 | (*R*)-3-CH₃ | H | 220-222 | AcOEt | I |
| 17 | 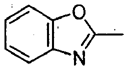 | (*S*)-3-CH₃ | H | 191-193 | AcOEt | I |
| 18 | 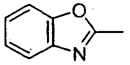 | cis-3-CH₃ | Cis-5-CH₃ | 165-167 | AcOEt | I |
| 19 | 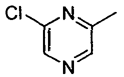 | (*R*)-3-CH₃ | H | 167-169 | i-PrOH | I |
| 20 | 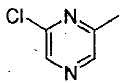 | (*S*)-3-CH₃ | H | 224-226 | EtOH | II |
| 21 | 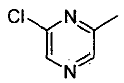 | cis-3-CH₃ | cis-5-CH₃ | 174-175 | EtOH | I |
| 22 | 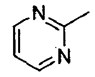 | (*R*)-3-CH₃ | H | 151-153 | (i-Pr)₂O | I |
| 23 | 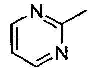 | (*S*)-3-CH₃ | H | 212-214 | AcOEt | II |
| 24 | 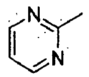 | cis-3-CH₃ | cis-5-CH₃ | 163-164 | i-PrOH + (i-Pr)₂O | I |
| 25 | 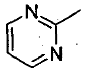 | trans-3-CH₃ | trans-5-CH₃ | 237-239 | i-PrOH | I |
| 26 | 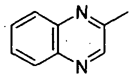 | (*S*)-3-CH₃ | H | 160-161 | AcOEt + (i-Pr)₂O | I |
| 27 | 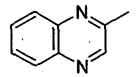 | cis-3-CH₃ | cis-5-CH₃ | 156-157 | AcOEt (i-Pr) ₂O | I |
| 28 | 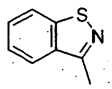 | (*S*)-3-CH₃ | H | 176-178 | EtOH + (i - Pr) ₂O | I |
| 29** | 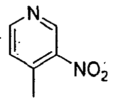 | cis-3-CH₃ | cis-5-CH₃ | amorphous | | III |
| 30 | 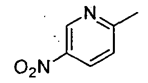 | cis-3-CH₃ | cis-5-CH₃ | 208-210 | AcOEt | I |
| 31 | 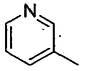 | (R)-3-CH₃ | H | 111-113 | (i-Pr)₂O | I |
| 32 | 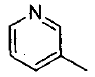 | (S)-3-CH₃ | H | 114-115 | (i-Pr)₂O | I |
| 33** | 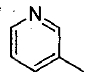 | cis-3-CH₃ | cis-5-CH₃ | oil | | I |
| 34 | 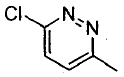 | (R)-3-CH₃ | H | 177-179 | i-PrOH | I |
| 35 | 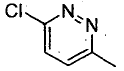 | (*S*)-3-CH₃ | H | 183-186 | AcOEt | II |
| 36 | 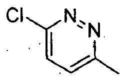 | cis-3-CH₃ | cis-5-CH₃ | 182-184 | i-PrOH | I |
| 37 | 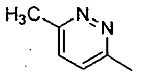 | (S)-3-CH₃ | H | 192-194 | AcOEt | I |
| 38 | 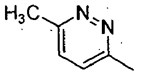 | cis-3-CH₃ | cis-5-CH₃ | 158-159 | i-PrOH + (i-Pr)₂O | I |
| 39 | 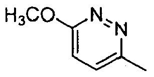 | cis-3-CH₃ | cis-5-CH₃ | 173-175 | i-PrOH + (i-Pr)₂O | I |
| 40 | 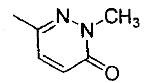 | (S)-3-CH₃ | H | 219-221 | i-PrOH + AcOEt | I |
| 41 | 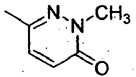 | cis-3-CH₃ | cis-5-CH₃ | 200-204 | (i-Pr) ₂O | |
| 55** | 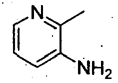 | cis-3-CH₃ | cis-5-CH₃ | amorphous | | |
| 56** | 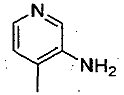 | cis-3-CH₃ | cis-5-CH₃ | amorphous | | |
| 71 | 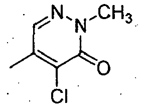 | (*S*)-3-CH₃ | H | 197-200 | EtOH | II |
| 72 | 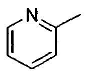 | (*S*)-3-CH₃- | H | 186-189 | EtOH | I |
| 73 | 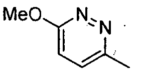 | (*S*)-3-CH₃ | H | 134-137 | i-PrOH + (i-Pr)₂O | I |
| 74 | 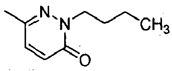 | (*S*)-3-CH₃ | H | 186-188 | EtOH | II^{*} |
| 75 | 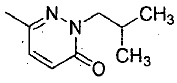 | (*S*)-3-CH₃ | H | 215-216 | EtOH | II^{*} |
| 76** | 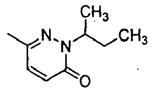 | (*S*)-3-CH₃ | H | amorphous | | II^{*} |
| 77 | 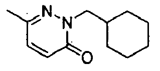 | (*S*)-3-CH₃ | H | 215-217 | ETOH | II^{*} |
| 78 | 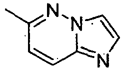 | (*S*)-3-CH₃ | H | 190-193 | AcOEt | I |
| 79 | 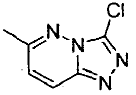 | (*S*)-3-CH₃ | H | 268-269 | EtOH + Et₂O | II^{*} |
| 80 | 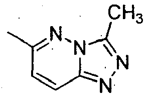 | (*S*)-3-CH₃ | H | 283-285 (sublime) | EtOH | II* |

| | | | | | | |
|---|---|---|---|---|---|---|
| *Sodium hexamethyldisilazide was used as an alternative to sodium tert-butoxide. **Example 29: ¹H NMR (CDCl₃) δ: 9.03 (1H, s), 8.50 (1H, d, J=5.9Hz), 7.5-6.8 (5H, m), 5.38-5.31 (1H, m), 4.43-4.40 (1H, m), 3.8-3.5 (2H, m), 3.21-3.17 (1H, m), 2.63-2.53 (1H, m), 2. 1-1. 5 (4H, m), 0. 97-0.77 (7H, m) Example 33: ¹H NMR (CDCl₃) δ: 8.49 (1H, dd, J=16.3, 2.7Hz), 8.23 (1H, d, =4.6Hz), 7.58-7.54. (1H, m), 7.26-7.22 (1H, m), 7.16-7.11 (3H, m), 6.84-6.79 (1H, m), 5.10-5.05 (1H, m), 4.54 (1H, d, J=12.9Hz), 3. 85-3.80, (1H, m), 3.66-3.57 (1H, m), 3.13-3.06 (1H, m), 2.57 (1H, t, J=12.6Hz), 2.04 (1H, q, J=12.0Hz), 1.84-1.41 (3H, m), 0.91-0.73.(7H, m). Example 55 : ¹H NMR (CDCl₃)δ: 7.82-7.79 (1H, m), 7.11-6.96. (4H, m), 6.72 (1 H, t, J=7.3Hz), 6.23 (1H, t, J=7.6Hz), 5.73-5.30 (1H, m), 4.6-4.3 (3H, m), 3.95-3.80 (1H, m), 3.59-3.42 (1H, m), 3.25-3.15 (1H, m), 2.57-2.49 (1H, m), 2.1-1.4 (5H, m), 0.95-0.73 (7H, m) Example 56: ¹H NMR (CDCl₃)δ: 8.19 (1H, s), 7.97 (1H, d, J=5.2Hz), 7.13-6.96 (3H, m), 6.76(1H, t, J=7.2Hz), 6.28(1H, dd, J=7.7, 13.5Hz), 5.14-5.09 (1H, m), 4.6-4.2 (3H, m), 3.8-3.5 (2H, m) 3.25-3.15 (1H, m), 2.56-2.45 (1H, m), 2.1-1.4 (5H, m), 0.96-0.72 (7H, m) Example 76: ¹H NMR (CDCl₃)δ: 7. 42-7.39 (1H, m), 7.3-7.1 (2H, m), 6.94(1H, d, J=10.0Hz), 6.9-6.8 (1H, m), 5.4-5.2 (1H, m), 5.1-5.0 (1H, m), 4.6-4.2 (1H, m); 4.0-3.7 (1H, m), 3.2-2.1 (3H, m), 2.0-0.7 (16H, m) | | | | | | |

**Table 5**

| | | | | | | |
|---|---|---|---|---|---|---|
| 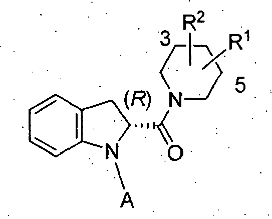 | | | | | | |

| Ex. | A | R¹ | R² | Melting point : (°C) | Recrystallizing Solvent | Preparation |
|---|---|---|---|---|---|---|
| 42 | 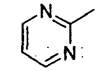 | cis-3-CH₃ | cis-5-CH₃ | 159-160 | (i-Pr)₂O | I |
| 81 | 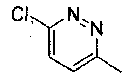 | (*S*)-3-CH₃ | H | 185-187 | EtOH | II |
| 82 | 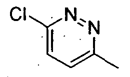 | (*R*)-3-CH₃ | H | 185-187 | EtOH | II |

### Example 43

### Preparation of 1-(2-pyrimidinyl)-2-(1-pyrrolidinylcarbonyl)indoline:

0.30 g of 1-(2-Pyrimidinyl)indoline-2-carboxylic acid and 0.088 g of pyrrolidine were dissolved in 5 ml of tetrahydrofuran, and thereto was added 0.18 g of 1-hydroxybenzotriazole. The mixture was cooled to 0°C, and then thereto was slowly added dropwise 0.21 g of 1-ethyl-3-(3-dimethylaminopropyl)carbodiimide and the mixture was stirred for 15 hours at room temperature. After evaporating tetrahydrofuran, the residue was dissolved in ethyl acetate and the solution was washed with 10% aqueous citric acid, water, saturated aqueous sodium hydrogen carbonate and brine. The solution was dried over anhydrous sodium sulfate and filtrated. The filtrate was concentrated in vacuo. The residue was purified by silica gel column chromatography eluted by hexane/ethyl acetate (3:1) and recrystallized from ethyl acetate to give 0.21g of the desired compound.
Melting point: 220-223°C

### Examples 44-54

1-(2-Pyrimidinyl)indoline-2-carboxylic acid was reacted and treated with various cyclic amines in the similar manner as Example 43 to give the compounds of Examples 44-54 in Table 6.

**Table 6**

| | | | | | | | |
|---|---|---|---|---|---|---|---|
| 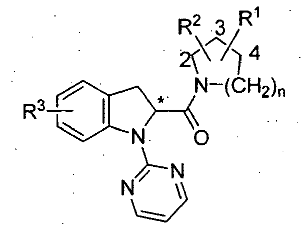 | | | | | | | |

| EX. | n | R¹ | R² | R³ | M.P. (°C) | Recrystallizing Solvent | Stereochemistry of 2 position of indoline ring |
|---|---|---|---|---|---|---|---|
| 43 | 1 | H | H | H | 220-223 | AcOEt | racemic |
| 44 | 2 | (*R*)-3-NHBoc | H | H | 261-263 | CH₃CN + MeOH | racemic |
| 45 | 2 | (*R*)-3-OH | H | H | 198-201 | (i-Pr)₂O | R* |
| 46 | 2 | (*R*)-3-OH | H | H | 189-192 | (i-Pr)₂O | 5* |
| 47 | 2 | (*S*)-3- NHBoc | H | H | 261-263 | CH₃CN + MeOH | racemic |
| 48 | 2 | 3-F | 3-F | H | 172-174 | (i-Pr)₂O | racemic |
| 49 | 2 | 3-CF₃ | H | H | 159-163 | (i-Pr)₂O | racemic |
| 50 | 2 | 3-CH₂OH | H | H | 124-128 | (i-Pr)₂O | racemic |
| 51 | 2 | 3-Ph | H | H | 145-151 | (i-Pr)₂O | racemic |
| 52 | 2 | 2-CH₃ | H | H | 166-168 | i-PrOH + (i-Pr)₂O | racemic |
| 53 | 2 | 4-CH₃ | H | H | 183-185 | AcOEt | racemic |
| 54 | 3 | H | H | H | 176-177 | i-PrOH | racemic |

### Example 55

### Preparation of (S)-1-(3-aminopyridin-2=yl.)-2-[(cis=3,5-dimethylpiperidin-1-yl)carbonyl]indoline:

0.6 g of (S)-2-[(cis-3,5-Dimethylpiperidin-1-yl)-carbonyl]-1-(3-nitropyridin-2-yl)indoline was dissolved in 10 ml of ethanol, and thereto was added 60 mg of 10% palladium carbon at 0°C. The mixture was stirred for 2 hours at room temperature under hydrogen atmosphere. The reaction mixture was filtered through Celite, and then the filtrate was concentrated in vacuo. The residue was purified by silica gel column chromatography eluted by hexane/ethyl acetate (3:1) to give 0.38 g of the amorphous desired compound.

### Example 56

### Preparation of (S)-1-(3-aminopyridin-4-yl)-2-[(cis-3,5-dimethylpiperidin-1-yl)carbonyl]indoline:

(S)-2-[(cis-3,5-Dimethylpiperidin-1-yl)carbonyl]-1-(3-nitropyridin-4-yl)indoline was reacted and treated in the similar manner as Example 55 to give the amorphous desired compound.

### Example 57

### Preparation of (S)-2-[(cis-3,5-dimethylpiperidin-1-yl)-carbonyl]-1-(3-pyridazinyl)indoline:

3.6 g of (S)-1-(6-Chloropyridazin-3-yl)-2-[(cis-3,5-dimethylpiperidin-1-yl)carbonyl]indoline was suspended in 50 ml of ethanol, and thereto were added 19..4 ml of 0.5 mol/l aqueous sodium hydroxide and 0.4 g of 10% palladium carbon at 0°C. The mixture was stirred for 4 hours at room temperature under hydrogen atmosphere. The reaction mixture was filtered through Celite, and then the filtrate was concentrated in vacuo. To the residue was added ethyl acetate, and the mixture was washed with water and brine. The ethyl acetate solution was dried over anhydrous sodium sulfate and was filtrated. The filtrate was concentrated in vacuo. The residue was purified by silica gel column chromatography eluted by chloroform/methanol (100:1) and recrystallized from isopropanol/diisopropyl ether to give 1.2 g of the desired compound. Melting point: 159-160°C

### Examples 58-63

The compounds of Examples 9, 19, 20, 21, 34 and 35 were reacted and treated in the similar manner as Example 57 to give the compounds of Examples 58-63 in Table 7.

**Table 7**

| | | | | | | | |
|---|---|---|---|---|---|---|---|
| 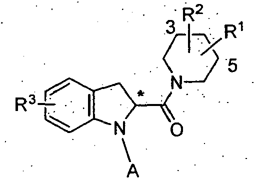 | | | | | | | |

| Ex. | A | R¹ | R² | R³ | M. P. (°C) | Recrystallizing Solvent | Stereo-chemistry of 2 position of indoline ring |
|---|---|---|---|---|---|---|---|
| 57 | 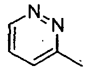 | cis-3-CH₃ | cis-5-CH₃ | H | 159-160 | (i-Pr)₂O i-PrOH | *S* |
| 58 | 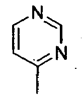 | cis-3-CH₃ | cis-5-CH₃ | H | 107-109 | Et₂O | racemic |
| 59 | 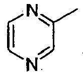 | (*R*) 3-CH₃ | H | H | 177-179 | AcOEt | *S* |
| 60 | 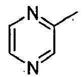 | (*S*)-3-CH₃ | H | H | 174-175 | AcOEt | *S* |
| 61 | 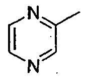 | cis-3-CH₃ | cis-5-CH₃ | H | 162-164 | i-PrOH + (i-Pr)₂O | *S* |
| 62 | 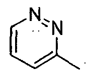 | (*R*)-3-CH₃ | H | H | 169-172 | (i-Pr)₂O + AcOEt | *S* |
| 63 | 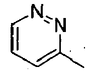 | (*S*)-3-CH₃ | H | H | 143-145 | (i-Pr)₂O + AcOEt | *S* |
| 83 | 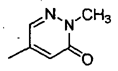 | (*S*)-3-CH₃ | H | H | 150-152 | i-PrOH | *S* |
| 84 | 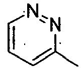 | (*S*)-3-CH₃ | H | 5-F | 155-157 | AcOEt | racemic |
| 85 | 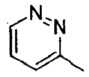 | (*S*)-3-CH₃ | H | 4-OCH₃ | 160-162 | (i-Pr)₂O | racemic |
| 86 | 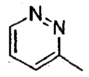 | (*S*)-3-CH₃ | H | 5-OCH ₃ | 136-139 | AcOEt | racemic |
| 87 | 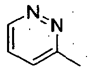 | (*S*)-3-CH₃ | H | 6-OCH ₃ | 139-141 | (i-Pr)₂O | racemic |
| 88 | 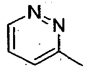 | (*S*)- 3-CH₃ | H | H | 167-168 | AcOEt + Et₂O | *R* |
| 89 | 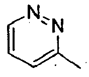 | (*R*) 3-CH₃ | H | H | 121-123 | AcOEt + Et₂O | *R* |

### Example 64

### Preparation of (S)-5-bromo-2-[(cis-3,5-dimethylpiperidirin-1-yl)carbonyl]-1-(2-pyrimidinyl)indoline:

0.5 g of (S)-2-[(cis-3,5-Dimethylpiperidin-1-yl)-carbonyl]-1-(2-pyrimidinyl)indoline was dissolved in 5 ml of dimethylformamide and the mixture was cooled to 0°C, and then thereto was added 0.26 g of N-bromosuccinimide. The mixture was stirred for 3 hours at 0°C, and then thereto was added water. The resulting crystal was filtrated and washed with water. The crystal was dried by heating, and then recrystallized from isopropanol to give 0.35 g of the desired compound.
Melting point: 142-144°C

### Example 65

### Preparation of 2-[[(R)-3-aminopiperidin-1-yl]carbonyl]-1-(2-pyrimidinyl)indoline dihydrochloride:

1.0 g of 2-[[(R)-3-(tert-Butoxycarbonylamino)-piperidin-1-yl]carbonyl]-1-(2-pyrimidinyl)indoline was dissolved in 5 ml of ethanol, and thereto was added 1 ml of 30% hydrochloric acid in ethanol and the mixture was stirred for 3 hours at room temperature. The reaction mixture was concentrated in vacuo, and then diethyl ether was added to the residue and the resulting crystal was filtrated. The crystal was recrystallized from ethanol to give 0.68 g of the desired compound.
Melting point: 205-209°C

### Example 66

### Preparation of 2-[[(S)-3-aminopiperidin-1-yl]carbonyl]-1-(2-pyrimidinyl)indoline dihydrochloride:

The compound of Example 47 was reacted and treated in the similar manner as Example 65 to give the desired compound.
Melting point: 207-210°C

### Examples 67-82

The compounds of Reference Examples 19, 27-30, 35 and 36 were reacted and treated with various halogenated heteroaromatic compounds in the similar manner as Example 1 (Preparation I) or Example 2 (Preparation II) to give the compounds of Examples 67-82 in Table 3, Table 4 and Table 5.

### Examples 83-89

The corresponding compounds were reacted and treated in the similar manner as Example 57 to give the compounds of Examples 83-89 in Table 7.

### Examples 90-94

The corresponding compounds were reacted and treated with N-bromosuccinimide or N-chlorosuccinimide in the similar manner as Example 64 to give the compounds of Examples 90-94 in Table 8.

**Table 8**

| | | | | | | | |
|---|---|---|---|---|---|---|---|
| 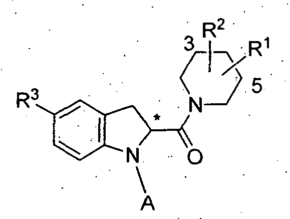 | | | | | | | |

| EX. | A | R¹ | R² | R³ | M.P. (°C) | Recrystallizing Solvent | Stereo-chemistry of 2 position of indoline ring |
|---|---|---|---|---|---|---|---|
| 64 | 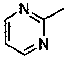 | cis-3-CH₃ | cis-5-CH₃ | Br | 142-144 | i-PrOH | *S* |
| 90 | 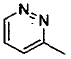 | (*S*)-3-CH₃ | H | Br | 161-163 | EtTOH | *S* |
| 91 | 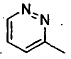 | (*S*) 3-CH₃ | H | Cl | 175-177 | EtOH | *S* |
| 92 | 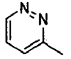 | (*S*)-3-CH₃ | H | Br | 237-240 | i-PrOH + (i-Pr)₂O | *R* |
| 93 | 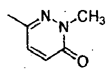 | (*S*)-CH₃ | H | Br | 241-242 | EtOH | *S* |
| 94 | 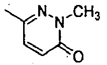 | (*S*)-3-CH₃ | H | Cl | 243-244 | EtOH | *S* |

### Example 95

### Preparation of (R)-2-[(S)-3-methylpiperidin-1-yl]carbonyl-1-(3-pyridazinyl)-5-vinylindoline:

2.0 g of (R)-5-Bromo-2-[(S)-3-methylpiperidin-1-yl]carbonyl-1-(3-pyridazinyl)indoline was dissolved in 30 ml of -1,2-dimethoxyethane, and thereto were added 1.0 g of 4,4,5,5-tetramethyl-2-vinyl-1,3,2-dioxaborolane, 0.17. g of tetrakis(triphenylphosphine)palladium and 7.5 ml of aqueous sodium carbonate (1 mol/l). The mixture was heated to reflux for 5 hours under nitrogen atmosphere. The reaction mixture was filtrated through Celite, and then to the filtrate was added 30 ml of ethyl acetate and the mixture was washed with water and brine. The ethyl acetate solution was dried over anhydrous sodium sulfate and was filtrated. The filtrate was concentrated in vacuo. The residue was purified by silica gel column chromatography eluted by chloroform/methanol (100:1) and recrystallized from ethyl acetate to give 0.81 g of the desired compound.
Melting point: 206-209°C

### Example 96

### Preparation of (S)-1-(1-inethyl-6-oxo-1,6-dihydropyridazin-3-yl)-2-[[(S)-3-methylpiperidin-1-yl]carbonyl]-5-phenyl-indoline:

The compound obtained in Example 93 was reacted and treated with phenylboronic acid in the similar manner as Example 95 to give the desired compound.
Melting point: 204-206°C

### Example 97

### Preparation of (R)-2-[(S)-3-methylpiperidin-1-yl]carbonyl-1-(3-pyridazinyl)-5-(1-piperidinyl)indoline:

700 mg of the compound obtained in Example 92, 190 mg of piperidine, 80 mg of tris(dibenzylideneacetone)-dipalladium, 220 mg of sodium tert-butoxide and 14 mg of tri-tert-butylphosphine were dissolved in anhydrous toluene under nitrogen atmosphere and the mixture was stirred for 5 hours at room temperature. The reaction mixture was filtered through Celite and the filtrate was concentrated in vacuo. The residue was purified by silica gel column chromatography' eluted by chloroform/methanol (100:1) and recrystallized from isopropanol to give 80 mg of the desired compound.
Melting point: 226-228°C

### Examples 98-103

The corresponding indoline derivatives were reacted and treated in the similar manner as Examples 95-97 to give the compounds of Examples 98-103 in Table 9.

**Table 9**

| | | | | | | | |
|---|---|---|---|---|---|---|---|
| 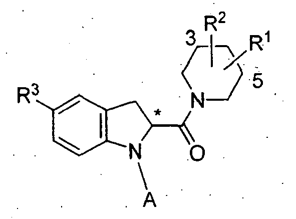 | | | | | | | |

| Ex. | A | R¹ | R² | R³ | M.P. (°C) | Recrystallizing Solvent | Stereo-chemistry of 2 position of indoline ring |
|---|---|---|---|---|---|---|---|
| 95 | 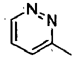 | (*S*)-3-CH₃ | H | vinyl | 206-209 | AcOEt | *R* |
| 96 | 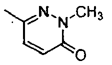 | (*S*)-3-CH₃ | H | Ph | 204-206 | EtOH | *S* |
| 97 | 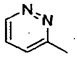 | (*S*)-3-CH₃ | H | 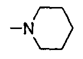 | 226-228 | i-PrOH | *R* |
| 98 | 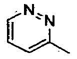 | (*S*)-3-CH₃ | H | vinyl | 103-106 | i-PrOH + (i-Pr)₂O | *S* |
| 99 | 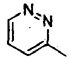 | (*S*)-3-CH₃ | H | N(C₂H₅)₂ | 148-150 | i-PrOH | *R* |
| 100 | 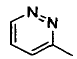 | (*S*)-3-CH₃ | H | NHCH₂Ph | 223-225 | AcOEt | *R* |
| 101 | 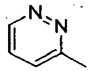 | (*S*)-3-CH₃ | H | N(C₂H₅)₂ | 161-165 | (i-Pr)₂O | *S* |
| 102 | 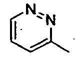 | (*S*)-3-CH₃ | H | 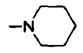 | 180-183 | AcOEt | *S* |
| 103 | 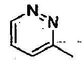 | (*S*)-3-CH₃ | H | NHCH₂Ph | 195-198 | i-PrOH | *S* |
| 104 | 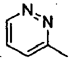 | (*S*)-3-CH₃ | H | C₂H₅ | 183-188 | AcOEt | *R* |
| 105 | 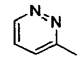 | (*S*)-3-CH₃ | H | C₂H₅ | 96-99 | (i-Pr)₂O | *S* |
| 106 | 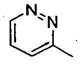 | (*S*)-3-CH₃ | H | NH₂ | 196-198 | i-PrOH | *S* |

### Example 104

### Preparation of (R)-5-ethyl-2-[(S)-3-methylpiperidin-1-yl]-carbonyl-1-(3-pyridazinyl)indoline:

1.0 g of the compound obtained in Example 95 was dissolved in 20 ml of ethanol, and thereto was added 0.1 g of 10% palladium carbon at 0°C. The mixture was stirred for 4 hours at room temperature under hydrogen atmosphere. The reaction mixture was filtrated through Celite, and then the filtrate was concentrated in vacuo. The residue was purified by silica gel column chromatography eluted by chloroform/methanol (100:1) and recrystallized from ethyl acetate to give 0.63 g of the desired compound.
Melting point: 183-188°C

### Example 105

### Preparation of (S)-5-ethyl-2-[(S)-3-methylpiperidin-1-yl]-carbonyl-1-(3-pyridazinyl)indoline:

The compound obtained in Example 98 was reacted and treated in the similar manner as Example 104 to give the desired compound.
Melting point: 96-99°C

### Example 106

### Preparation of (S)-5-amino-2-[(S)-3-methylpiperidin-1-yl]-carbonyl-1-(3-pyridazinyl)indoline:

The compound obtained in Example 103 was reacted and treated in the similar manner as Example 104 using 20% palladium carbon hydroxide by which 10% palladium carbon replaced as a catalyst, to give the desired compound.
Melting point: 196-198°C

### Example 107

### Preparation of (S)-1-(1-methyl-6-oxo-1,6-dihydropyridazin-3-yl)-2-(1-piperidinylcarbonyl)indoline:

To a suspension of 0.1 g of (S)-1-(1-methyl-6-oxo-1,6-dihydropyridazin-3-yl)indoline-2-carboxylic acid in 2.0 ml of tetrahydrofuran was added 76 mg of CDI (1,1'-carbonylbis-1H-imidazole), and then the mixture was stirred for 30 minutes at room temperature. 39 mg of Piperidine was added dropwise thereto at room temperature and the mixture was stirred for 12 hours. 2.0 ml of Water was added thereto and the mixture was ice-cooled. The resulting precipitate was filtrated, washed with water and dried. The precipitate was recrystallized from acetonitrile to give 30 mg of the desired compound.

### Examples 108-122

1-(1-Methyl-6-oxo-1,6-dihydropyridazin-3-yl)indoline-2-carboxylic acid was reacted and treated with various cyclic amines in the similar manner as Example 107 to give the compounds of Examples 108-122 in Table 10.

**Table 10**

| | | | | | | |
|---|---|---|---|---|---|---|
| 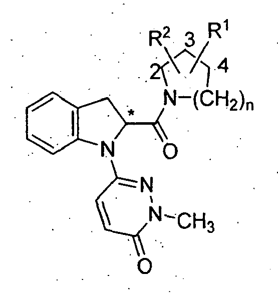 | | | | | | |

| Ex. | n | R¹ | R² | M.P. (°C) | Recrystallizing Solvent | Stereo-chemistry of 2 position of indoline ring |
|---|---|---|---|---|---|---|
| 107 | 2 | H | H | 225-227 | CH₃CN | *S* |
| 108* | 2 | 3-CF₃ | H | oil | | *S* |
| 109 | 2 | 3-CH₂OH | H | 182-185 | EtOH + H₂O | *S* |
| 110* | 2 | 4-CH₃ | H | oil | | *S* |
| 111 | 2 | 4-CH₂CH₃ | H | 204-208 | (i-Pr)₂O | *S* |
| 112 | 2 | 4-NHAc | H | 213-216 | (i-Pr)₂O | *S* |
| 113 | 1 | H | H | 224-229 | EtOH + H₂O | *S* |
| 114 | 1 | 3-NHAc | H | 194-198 | (i-Pr)₂O + CH₃COCH₃ | *S* |
| 115 | 1 | 3-NMe₂ | H | 192-195 | CH₃CN + (i-Pr)₂O | *S* |
| 116 | 1 | 3-N(CH₃)Ac | H | 91-95 | (i-Pr)₂O | *S* |
| 117 | 0 | 3-F | 3-F | 204-207 | (i-Pr)₂O | *S* |
| 118 | 3 | H | H | 198-199 | ETOH + H₂O | *S* |
| 119 | 4 | H | H | 179-182 | EtOH + H₂O | *S* |
| 120 | 2 | (S)-3-CH₃ | H | 192-194 | EtOH | *R* |
| 121 | 2 | (R)-3-CH₃ | H | 234-235 | EtOH | *R* |
| 122 | 2 | (R)-3-CH₃ | H | 191-193 | EtOH + (i-Pr)₂O | *S* |

| | | | | | | |
|---|---|---|---|---|---|---|
| ^{*}Example 108: ¹H NMR (CDCl₃)δ: 7.55-7.35 (1H, m), 7.3-7.1 (3H, m),7.0-6.8 (2H, m), 5.4-5.2 (1H, m), 4.7-4.3 (1H, m), 4.1-3.8 (1H, m), 3.7-3.5 (1H, m), 3.66 (3H, s), 3.3-3.0 (2H, m), 2.9-2.6 (1H, m), 2.3-1.1 (5H, m) Example 110: ¹H NMR (C_{D}Cl₃)δ: 7.5-7.1 (4H, m), 7.0-6.8 (2H., m), 5.3-5.2 (1H, m), 4.52 (1H, t, J=11.4Hz), 3.93 (1H, d, J = 13.4Hz), 3.68 (3H., s), 3.6-3.4 (1H, m), 3.3-3.0 (2H, m), 2.7-2.5 (1H, m), 1.9-1.7 (3H, m), 1.4-0.9 (5H, m) | | | | | | |

### Example 123

### Preparation of (S)-1-(1-benzyl-6-oxo-1, 6-dihydropyridazin-3-yl)-2-[[(S)-3-methylpiperidin-1-yl]carbonyl]indoline:

0.2 g of (S)-1-(1-Benzyl-6-oxo-1,6-dihydropyridazin-3-yl)indoline-2-carboxylic acid was dissolved in 5 ml of pyridine, and thereto were added 0.070 g of (S)-3-methylpiperidine and 0.13 g of 1-ethyl-3-(3-dimethylaminopropyl)carbodiimide hydrochloride and the mixture was stirred for 15 hours at room temperature. After completion of the reaction, the reaction mixture was poured into 30 ml of ice water and then the precipitate was filtrated. The resulting precipitate was washed with water and dried, and then the resulting precipitate was recrystallized from ethanol to give 0.090 g of the desired compound.
Melting point: 183-18.8°C

### Examples 124-128

The various indoline-2-carboxylic acids substituted on the 1-position which were obtained in Reference Examples 52-56 were reacted and treated with (S)-3-methylpiperidine in the similar manner as Example 123 to give the compounds of Examples 124-128 in Table 11.

**Table 11**

| | | | |
|---|---|---|---|
| 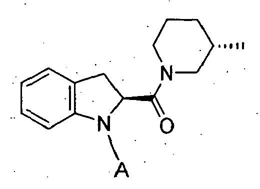 | | | |

| Ex. | A | M.P. (°C) | Recrystallizing Solvent |
|---|---|---|---|
| 123 | 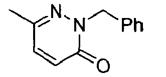 | 220-222 | EtOH |
| 124 | 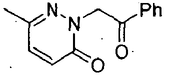 | 255(dec.) | DMF-H₂O |
| 125 | 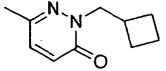 | 221-223 | DMF-H₂O |
| 126^{*} | 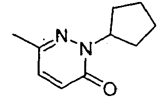 | amorphous | |
| 127 | 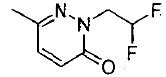 | 173-175 | EtOH |
| 128 | 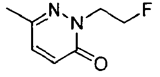 | 229-230 (sublime) | ETOH |

| | | | |
|---|---|---|---|
| *Example 126 : ¹H NMR (CDCl₃)δ: 7.5-7.4 (1H, m), 7.3-7.1 (3H, m), 7.0-6.8 (2H, m) 5.5-5.2 (2H, m), 4.6-4.2 (1H, m), 4.0-3.4 (2H, m), 3.2-2.3 (3H, m), 2.1-1.3 (12H, m), 1.3-1.0 (1H, m), 1.0-0.8 (3H, m) | | | |

### Example 129

### Preparation of (S)-1-(1-ethyl-6-.oxo-1,6-dihydropyridazin-3-yl)-2-[[(S)-3-methylpiperidin-1yl]carbonyl]indoline:

2.0 g of the compound obtained in Example 35 was dissolved in 30 ml of tetrahydrofuran, and thereto was added dropwise 11 ml of diethyl sulfate at 0°C. The mixture was stirred for 14 hours at room temperature, and then the resulting crystal was filtrated. The crystal was suspended in 50 ml of water, and thereto was added 17 ml,of

1 mol/l aqueous sodium hydroxide at 0°C and the mixture was stirred for 1 hour at room temperature. The reaction mixture was extracted with chloroform and the organic layer was washed with water and brine, dried over anhydrous sodium sulfate and was filtrated. The filtrate was concentrated in vacuo. The residue was purified by silica gel column chromatography eluted by chloroform/methanol (100:1) and recrystallized from ethanol to give 0.25 g of the desired compound.
Melting point: 197-199°C

### Examples 130-135

The corresponding 1-(6-chloropyridazin-3-yl)indolines were reacted and treated with dimethyl sulfate, dipropyl sulfate or 1,3,2-dioxathiolane-2,2-dioxide in the similar manner as Example 129 to give the compounds of Examples 130-135 in Table 12.

**Table 12**

| | | | | | |
|---|---|---|---|---|---|
| 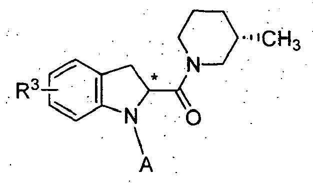 | | | | | |

| Ex. | A | R³ | M.P. (°C) | Recrystallizing Solvent | Stereo-chemistry of 2 position of indoline ring |
|---|---|---|---|---|---|
| 129 | 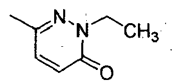 | H | 197-199 | EtOH | *S* |
| 130 | 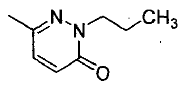 | H | 183-185 | EtOH | *S* |
| 131 | 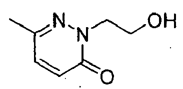 | H | 201-204 | AcOEt | *S* |
| 132 | 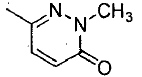 | 5-F | 217-219 | EtOH | racemic |
| 133 | 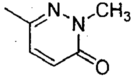 | 4-OCH₃ | 181-182 | i-PrOH | racemic |
| 134 | 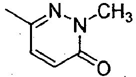 | 5-OCH₃ | 248-250 | EtOH | racemic |
| 135 | 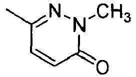 | 6-OCH₃ | 182-184 | EtOH | racemic |
| 136 | 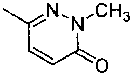 | 5-OH | 253-256 | EtOH | racemic |
| 137 | 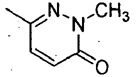 | 4-OH | 297-299 | EtOH | racemic |
| 138 | 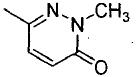 | 6-OH | 259-261 | EtOH | racemic |
| 139 | 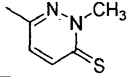 | H | 249-252 | CH3CN + MeOH | *S* |

### Example 136

### Preparation of 5-hydroxy-1-(1-methyl-6-oxo-1,6-dihydropyridazin-3-yl)-2-[[(S)-3-methylpiperidin-1-yl]carbonyl]indoline:

0.2 g of the compound obtained in Example 134 was dissolved in 5 ml of dichloromethane, and thereto was added dropwise 3.2 ml of a solution of boron tribromide in dichloromethane (1 mol/1) at 0°C. The mixture was stirred for 6 hours at room temperature and then the reaction mixture was added to 20 ml of ice water. The mixture was allowed to be neutral with aqueous sodium hydroxide, and then was extracted with dichloromethane. The extract was washed with water and brine, dried over anhydrous sodium sulfate and filtrated. The filtrate was concentrated in vacuo. The residue was purified by silica gel column chromatography eluted by chloroform/methanol (50:1) and recrystallized from ethanol to give 58 mg of the desired compound.
Melting point: 253-256°C

### Examples 137-138

The compounds obtained in Example 133 and Example 135 were reacted, treated and purified in the similar manner as Example 136 to give the compounds of Example 137 and Example 138 in Table 12.

### Example 139

### Preparation of (S)-2-[[(S)-3-methylpiperidin-1-yl]carbonyl]-1-(1-methyl-6-thioxo-1,6-dihydropyridazin-3-yl)indoline:

0.8 g of the compound obtained in Example 35 was dissolved in 15 ml of tetrahydrofuran, and thereto was added dropwise 3.2 ml of dimethyl sulfate at 0°C. The mixture was stirred for 14 hours at room temperature and then the resulting crystal was filtrated. The crystal was suspended in 10 ml of dichloromethane, and thereto was added 0.16 g of thiourea and the mixture was heated to reflux for 2 hours. The mixture was cooled to room temperature, and then thereto was added 10 ml of ammonia water and the mixture was stirred for 30 minutes. The mixture was extracted with dichloromethane. The extract was washed with water and brine, dried over anhydrous sodium sulfate and was filtrated. The filtrate was concentrated in vacuo. The residue was purified by silica gel column chromatography eluted by chloroform/methanol (100:1) and recrystallized from ethanol-acetonitrile to give 0.28 g of the desired compound.
Melting point: 249-252°C

### Example 140

### Preparation of 1-(6-chloropyridazin-3-yl)-3-methyl-2-[[(S)-3-methylpiperidin-1-yl]carbonyl]indoline:

The compound obtained in Reference Example 37 was reacted and treated with 3,6-dichloropyridazine in the similar manner as Example 2 and recrystallized from diisopropyl ether to give the desired compound.
Melting point: 169-171°C

### Example 141

### Preparation of 3-methyl-1-(1-methyl-6-oxo-1,6-dihydropyridazin-3-yl)-2-[[(S)-3-methylpiperidin-1-yl]carbonyl]indoline:

The compound obtained in Example 140 was reacted and treated with dimethyl sulfate in the similar manner as Example 129 and recrystallized from diisopropyl ether to give the desired compound.
Melting point: 165-170°C

### Example 142

### Preparation of 2-methyl-1-(1-methyl-6-oxo-1,6-dihydro-pyridazin-3-yl)-2-[[(S)-3-methylpiperidin-1-yl]carbonyl]-indoline:

To a solution of 0.34 g of the compound obtained in Reference Example 58 in 10 ml of 1,4-dioxane were added 148 mg of (S)-3-methylpiperidine and 0.45 g of BBDI (1-t-butoxy-2-t-butoxycarbonyl-1,2-dihydroisoquinoline), and the mixture was heated to reflux for 2 hours. The reaction mixture was concentrated and the residue-was purified by silica gel column chromatography eluted by chloroform/methanol (50:1) to give 12 mg of the desired compound as amorphous.
¹H NMR (CDCl₃) δ: 7.8-7.5 (1H, m), 7.3-7.1 (3H, m), 7.1-6.8 (2H, m), 4.7-4.3 (1H, m), 3.8-3.4 (2H, m), 3.74 (3H, s), 3.2-2.2 (3H, m), 1.8-1.2 (7H, m), 1.1-0.8 (4H, m)

### Example 143

### Preparation of (S)-2-[[(S)-3-methylpiperidin-1-yl]carbonyl]-1-(6-oxo-1,6-dihydropyridazin-3-yl)indoline:

0.25 g of (S)-1-(6-Methoxypyridazin-3-yl)-2-[[(S)-3-methylpiperidin-1-yl]carbonyl]indoline obtained in Example 73 was dissolved in 5 ml of dichloromethane, and thereto was added dropwise 4.3 ml of a solution of boron tribromide in dichloromethane (1 mol/l) at 0°C. The mixture was stirred for 12 hours at room temperature, and then the reaction mixture was added to 20 ml of ice water. The mixture was allowed to be alkaline (pH 12) with aqueous sodium hydroxide and washed with dichloromethane. Then, saturated aqueous ammonium chloride was added to the aqueous layer and the product was extracted with chloroform. The chloroform layer was washed with water and brine, dried over anhydrous sodium sulfate and filtrated. The filtrate was concentrated in vacuo. The residue was recrystallized from diisopropyl ether to give 30 mg of the desired compound.
Melting point: 228-231°C

### Example 144

### Preparation of (S)-1-(1-methyl-6-oxo-1,6-dihydropyridazin-3-yl)-2-[[(S)-3-methylpiperidin-1-yl]carbonyl]indoline:

The compound obtained in Reference Example 40 was reacted and treated with-(S.)-3-methylpiperidine in the similar manner as Example 107 and recrystallized from ethanol to give the desired compound.
Melting point: 231-233°C

### Example 145

### Preparation of (S)-1-(1-methyl-6-oxo-1,6-dihydropyridazin-3-yl)-2-[(2-methylpiperidin-1-yl)carbonyl]indoline:

The compound obtained in Reference Example 40 was reacted and treated with 2-methylpiperidine in the similar manner as Example 142 and recrystallized from diisopropyl ether to give the desired compound.
Melting point: 96-98°C

### Example 146

### Preparation of (S)-2-[(3-ethylpiperidin-1-yl)carbonyl]-1-(1-methyl-6-oxo-1,6-dihydropyridazin-3-yl)indoline:

The compound obtained in Reference Example 40 was reacted and treated with 3-ethylpiperidine in the similar manner as Example 142 and recrystallized from diisopropyl ether to give the desired compound.
Melting point: 150-154°C

### Example 147

### Preparation of (S)-1-(1-methyl-6-oxo-1,6-dihydropyridazin-3-yl)-2-[(3-propylpiperidin-1-yl)carbonyl]indoline:

The compound obtained in Reference Example 40 was reacted and treated with 3-propylpiperidine in the similar manner as Example 142 and recrystallized from diisopropyl ether to give the desired compound.
Melting point: 106-110°C.

### Example 148

### Preparation of (S)-1-(1-methyl-6-oxo-1,6-dihydropyridazin-3-yl)-2-[(3-methylpyrrolidin-1-yl)carbonyl]indoline:

The compound obtained in Reference Example 40 was reacted and treated with 3-methylpyrrolidine in the similar manner as Example 142 and recrystallized from diisopropyl ether to give the desired compound.
Melting point: 150-154°C

### Example 149

### Preparation of (S)-2-[(3-ethylpyrrolidin-1-yl)carbonyl]-1-(1-methyl-6-oxo-1,6-dihydropyridazin-3-yl)indoline:

The compound obtained in Reference Example 40 was reacted and treated with 3-ethylpyrrolidine in the similar manner as Example 142 and recrystallized from diisopropyl ether to give the desired compound.
Melting point: 170-174°C

### Example 150

### Preparation of (S)-1-(1-methyl-6-oxo-1,6-dihydropyridazin-3-yl)-2-[(3-methylperhydroazepin-1-yl)carbonyl]indoline:

The compound obtained in Reference Example 40 was reacted and treated with 3-methylperhydroazepine in the similar manner as Example 142 and recrystallized from diisopropyl ether to give the desired compound.
Melting point: 109-112°C

### Example 151

### Preparation of (S)-2-[(3-ethylperhydroazepin-1-yl)carbonyl]-1-(1-methyl-6-oxo-1,6-dihydropyridazin-3-yl)indoline:

The compound obtained in Reference Example 40 was reacted and treated with 3-ethylperhydroazepine in the similar manner as Example 142 and recrystallized from diisopropyl ether to give the desired compound.
Melting point: 90-95°C

### Example 152

### Preparation of (S)-2-[(3,3-dimethylpiperidin-1-yl)carbonyl]-1-(1-methyl-6-oxo-1,6-dihydropyridazin-3-yl)indoline:

The compound obtained in Reference Example 40 was reacted and treated with 3,3-dimethylpiperidine in the similar manner as Example 142 and recrystallized from diisopropyl ether to give the desired compound.
Melting point: 125-129°C

### Example 153

### Preparation of (S)-2-[(S)-3-methylpiperidin-1-yl]carbonyl-1-(1,3-thiazol-2-yl)indoline:

The compound obtained in Reference Example 19 was reacted and treated with 2-bromothiazole in the similar manner as Example 1 and recrystallized from ethanol to give the desired compound.
Melting point:167-169°C

### Example 154

### Preparation of (S)-2-[[(S)-3-methylpiperidin-1-yl]carbonyl]-1-(1,2,4-triazolo[4,3-b]pyridazin-6-yl)indoline:

The compound obtained in Example 79 was reacted and treated in the similar manner as Example 57 and recrystallized from ethanol to give the desired compound. Melting point: >300°C

### Example 155

### Preparation of 4-fluoro-1-(1-methyl-6-oxo-1,6-dihydro-pyridazin-3-yl)-2-[[(S)-3-methylpiperidin-1-yl]carbonyl]-indoline:

(1) 4-Fluoroindoline-2-carboxylic acid was reacted and treated in the similar manner as Reference Example 38 to give 1-(6-chloropyridazin-3-yl)-4-fluoroindoline-2-carboxylic acid.
(2) 1-(6-chloropyridazin-3-yl)-4-fluoroindoline-2-carboxylic acid was reacted and treated in the similar manner as Reference Example 40 to give 4-fluoro-1-(1-methyl-6-oxo-1,6-dihydropyridazin-3-yl)indoline-2-carboxylic acid.
(3) 4-Fluoro-1-(1-methyl-6-oxo-1,6-dihydropyridazin-3-yl)indoline-2-carboxylic acid was reacted and treated with (S)-3-methylpiperidine-in the similar manner as Example 107 and recrystallized from diisopropyl ether/isopropanol to give the desired compound as a diastereomixture (2R:2S=1:4, a compound obtained in Example 155a; Melting point: 197-199°C). Additionally, after concentrating the mother liquid, the residue was recrystallized from diisopropyl ether to give the desired compound as a diastereomixture (2R:2S=3:2, a compound obtained in Example 155b; Melting point: 174-175°C).

### Example 156

### Preparation of 4-fluoro-1-(1-methyl-6-oxo-1,6-dihydro-pyridazin-3-yl)-2-[(1-perhydroazepinyl)carbonyl]indoline:

4-Fluoro-1-(1-methyl-6-oxo-1,6-dihydropyridazin-3-yl)indoline-2-carboxylic acid was reacted and treated with hexamethylene imine in the similar manner as Example 107 and recrystallized from ethyl acetate to give the desired compound.
Melting point: 211-213°C

### Example 157

### Preparation of 6-fluoro-1-(1-methyl-6-oxo-1,6-dihydro-pyridazin-3-yl)-2-[[(S)-3-methylplperidin-1-yl]carbonyl]-indoline:

(1) 6-Fluoroindoline-2-carboxylic acid was reacted and treated in the similar manner as Reference Example 38 to give-1-(6-chloropyridazin-3-yl)-6-fluoroindoline-2-carboxylic acid.
(2) 1-(6-Chloropyridazin-3-yl)-6-fluoroindoline-2-carboxylic acid was reacted and treated in the similar manner as Reference Example 40 to give 6-fluoro-1-(1-methyl-6-oxo-1,6-dihydropyridazin-3-yl)indoline-2-carboxylic acid.
(3) 6-Fluoro-1-(1-methyl-6-oxo-1,6-dihydropyridazin-3-yl)indoline-2-carboxylic acid was reacted and treated with (S)-3-methylpiperidine in the similar manner as Example 107 and recrystallized from isopropanol to give the desired compound.
Melting point: 219-222°C

### Example 158

### Preparation of 6-fluoro-1-(1-methyl-6-oxo-1,6-dihydro-pyridazin-3-yl)-2-[(1-perhydroazepinyl)carbonyl]indoline:

6-Fluoro-1-(1-methyl-6-oxo-1,6-dihydropyridazin-3-yl)indoline-2-carboxylic acid was reacted and treated with hexamethylene imine in the similar manner as Example 107 and recrystallized from isopropanol to give the desired compound.
Melting point: 204-207°C

### Example 159

### Preparation of cis-3-hydroxy-1-(1-methyl-6-oxo-1,6-dihydro-pyridazin-3-yl)-2-[(3-methylpiperidin-1-yl)carbonyl] indoline:

400 g of the compound obtained in Example 35 was reacted and treated with 1570 ml of dimethyl sulfate in the similar manner as Example 129. The residue was subjected to a silica gel column chromatography eluted by chloroform/methanol (100:1) to give 1.5 g of a crude crystal of the desired compound and 345 g of the compound obtained in Example 40. The crude crystal of the desired compound was subjected to a silica gel column chromatography eluted by chloroform/methanol (50:1) again for purification. The product was recrystallized from ethanol twice to give 5.0 mg of the desired compound. Melting point: 274-277°C

### INDUSTRIAL APPLICABILITY

As mentioned above, the compounds of formula (I) and pharmaceutically acceptable acid addition salts thereof exhibit a selective and strong affinity for MBR and additionally exhibit a potent pharmacological effect such as antianxiety effect and antidepressant effect in the animal tests. Therefore the compounds of the present invention will be useful as a medicament for treating/preventing anxiety disorders (panic disorder, generalized anxiety disorder, social-anxiety disorder, obsessive-compulsive disorder, posttraumatic stress disorder and so on), depressions /mood disorder, epilepsy, dementia (Alzheimer's disease, cerebrovascular dementia and so on), anxiety and depression, sleep disorder, nervous disease(Huntington's disease, multiple sclerosis, peripheral nerve disease and so**·**on), stress-related gastrointestinal disorders(stomach and duodenal ulcer, irritable bowel syndrome and so on), inflammatory disease(rheumatoid arthritis and so on), and cancer.

## Claims

1. A 2-(cyclic aminocarbonyl)indoline derivative of the following formula (I): 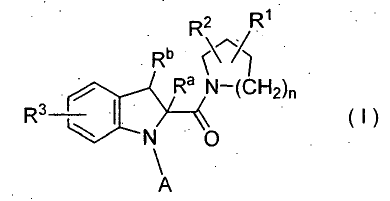 wherein
A is a group of the following formula (I-A): 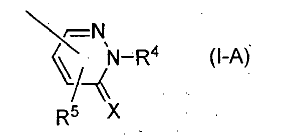 wherein X is oxygen atom or sulfur atom, R⁴ is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, a hydroxy-C₁₋₆ alkyl group, or a mono- or di-fluoro-C₁₋₆ alkyl group, R⁵ is hydrogen atom or a halogen atom, or
a heteroaryl group which is a 5- or a 6-membered monocyclic or fused polycyclic aromatic heteroaryl group containing 1-4 hetero atoms selected from the group consisting of N, O, and S, wherein the heteroaryl group may be optionally substituted with a halogen atom, a C₁₋₆ alkyl group, a C₁₋₆ alkoxy group, nitro group or amino group;
R¹ and R² are the same or different and are hydrogen atom, a halogen atom, a C₁₋₆ alkyl group, trifluoromethyl group, a hydroxy-C₁₋₆ alkyl group, hydroxy group, amino group, a di (C₁₋₆ alkyl) amino group, a C₁₋₆ alkylcarbonylamino group, a (C₁₋₆ alkyl) (C₁₋₆ alkylcarbonyl)amino group, a C₁₋₆ alkyloxycarbonyl-amino group or an aryl group;
R³ is hydrogen atom, a halogen atom, a C₁₋₆ alkyl group, a C₂₋₆ alkenyl group, a C₁₋₆ alkoxy group, a hydroxy group, an amino group, a di (C₁₋₆ alkyl)amino group, 1-pyrrolidinyl group, 1-piperidinyl group, an aryl-C₁₋₆ alkylamino group or an aryl group;
R^{a} and R^{b} are the same or different and are hydrogen atom or a C₁₋₆ alkyl group; and
n is an integer of 0-5,
or a pharmaceutically acceptable acid addition salt thereof.

2. The 2-(cyclic aminocarbonyl)indoline derivative or a pharmaceutically acceptable acid addition salt thereof according to claim 1,
wherein R¹ and R² are the same or different and are hydrogen atom, a C₁₋₆ alkyl group or trifluoromethyl group;
R³ is hydrogen atom, a halogen atom, a C₁₋₆ alkyl group, a C₂₋₆ alkenyl group or a C₁₋₆ alkoxy group; and
R^{a} is hydrogen atom.

3. The 2-(cyclic aminocarbonyl)indoline derivative or a pharmaceutically acceptable acid addition salt thereof according to claim 1,
wherein
A is a group of the following formula (I-A1): 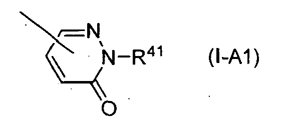 wherein R⁴¹ is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, or a mono- or a di-fluoro-C₁₋₆ alkyl group, or
furyl group, thienyl group, oxazolyl group, isoxazolyl group, thiazolyl group, isothiazolyl group, pyridyl group, pyrazinyl group, pyrimidinyl group, pyridazinyl group, benzoxazolyl group, benzisoxazolyl group, benzothiazolyl group, benzisothiazolyl group, quinolyl group, isoquinolyl group, quinoxalinyl group, quinazolinyl group, phthalazinyl group, cinnolinyl group, naphthyridinyl group, imidazopyridazinyl group or triazolopyridazinyl group, wherein each group may be optionally substituted with a halogen atom, a C₁₋₆ alkyl group or a C₁₋₆ alkoxy group;
R¹ and R² are the same or different and are hydrogen atom, a C₁₋₆ alkyl group or trifluoromethyl group;
R³ is hydrogen atom, a halogen atom, a C₁₋₆ alkyl group, a C₂₋₆ alkenyl group or a C₁₋₆ alkoxy group;
R^{a} is hydrogen atom;
R^{b} is hydrogen atom or a C₁₋₆ alkyl group; and
n is an integer of 1-4.

4. The 2-(cyclic aminocarbonyl)indoline derivative or a pharmaceutically acceptable acid addition salt thereof according to claim 1,
wherein
A is a group of the following formula (I-A2): 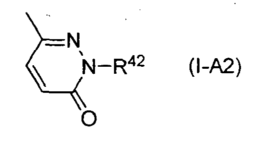 wherein R⁴² is hydrogen atom, a C₁₋₆ alkyl group, a C₃₋₆ cycloalkyl group, a C₃₋₆ cycloalkyl-C₁₋₆ alkyl group, an aryl-C₁₋₆ alkyl group, an arylcarbonyl-C₁₋₆ alkyl group, or a mono- or di-fluoro-C₁₋₆ alkyl group, or
a group of the following formula (I-A3) : 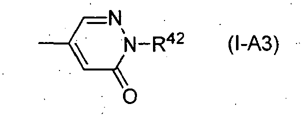 wherein R⁴² is as defined above;
R¹ and R² are the same or different and are hydrogen atom, a C₁₋₆ alkyl group or trifluoromethyl group;
R³ is hydrogen atom, halogen atom or a C₁₋₆ alkoxy group;
R^{a} is hydrogen atom;
R^{b} is hydrogen atom or methyl group; and
n is an integer of 1-4.

5. The 2-(cyclic aminocarbonyl)indoline derivative according to claim 1, which has the following formula (Ia): 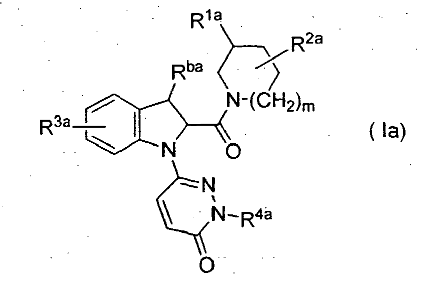 wherein
R^{1a} is hydrogen atom, methyl group, ethyl group, propyl group or trifluoromethyl group, which is bound on the 3-positions of the cyclic amino group;
R^{2a} is hydrogen atom, or methyl group, which is bound on the 3- or 5-positions of the cyclic amino group;
R^{3a} is hydrogen atom, fluorine atom, chlorine atom, bromine atom, or methoxy group, which is bound on the 4-, 5-, or 6-position of the indoline ring;
R^{4a} is hydrogen atom, methyl group, ethyl group, propyl group, isopropyl group, butyl group, isobutyl group, sec-butyl group, cyclopentyl group, cyclohexylmethyl group, benzyl group, phenylcarbonylmethyl group, 2-fluoroethyl group or 2,2-difluoroethyl group;
R^{ba} is hydrogen atom or methyl group; and
m is 0, 1, 2 or 3,
or a pharmaceutically acceptable acid addition salt thereof.

6. The 2-(cyclic aminocarbonyl)indoline derivative or a pharmaceutically acceptable acid addition salt thereof according to claim 1,
wherein
A is thiazolyl group, pyridyl group, pyrazinyl group, pyrimidinyl group, pyridazinyl group, 1,3-benzoxazolyl group, 1,3-benzothiazolyl group, 1,2-benzisothiazolyl group, isoquinolyl group, quinoxalinyl group, imidazo[1,2-b]pyridazinyl group, or 1,2,4-triazolo[4,3-b]pyridazinyl group, wherein each group may be optionally substituted with a halogen atom, a C₁₋₆ alkyl group or a C₁₋₆ alkoxy group;
R¹ and R² are the same or different and are hydrogen atom, a C₁₋₆ alkyl group or trifluoromethyl group;
R³ is hydrogen atom, a halogen atom or a C₁₋₆ alkoxy group;
R^{a} is hydrogen atom;
R^{b} is hydrogen atom or methyl group; and
n is an integer of 1-4.

7. The 2-(cyclic aminocarbonyl)indoline derivative or a pharmaceutically acceptable acid addition salt thereof according to claim 1,
wherein
A is 2-thiazolyl group, 2-pyridyl group, 3-pyridyl group, 4-pyridyl group, 2-pyrazinyl group, 2-pyrimidinyl group, 4-pyrimidinyl group, 3-pyridazinyl group, 1,3-benzoxazol-2-yl group, 1,3-berizothiazol-2-yl group, 1,2-benzisothiazol-3-yl group, 1-isoquinolyl group, 2-quinoxalinyl group, or 1,2,4-triazolo[4,3-b]pyridazin-6-yl group, wherein each group may be optionally substituted with fluorine atom, chlorine atom, methyl group, ethyl group or methoxy group;
R¹ and R² are the same or different and are hydrogen atom, methyl group or trifluoromethyl group, wherein R¹ bound on the 3-positions of the cyclic amino group, and R² bound on the 3- or 5-position of the cyclic amino group;
R³ is hydrogen atom, fluorine atom, chlorine atom, bromine atom, or methoxy group, which is bound on the 4-, 5-, or 6-position of the indoline ring;
R^{a} is hydrogen atom;
R^{b} is hydrogen atom or methyl group; and
n is 1, 2, 3, or 4.

8. A 2-(cyclic aminocarbonyl)indoline derivative or a pharmaceutically acceptable acid addition salt thereof according to claim 1, wherein the configuration of the 2-positioned asymmetric carbon in the indoline ring is (S).

9. The 2-(cyclic aminocarbonyl)indoline derivative according to claim 1 which is selected from
(S)-1-(1,3-benzoxazol-2-yl)-2-[[(S)-3-methylpiperidin-1-yl]carbonyl]indoline,
(S)-2-[(S)-3-methylpiperidin-1-yl]carbonyl-1-(2-pyrimidinyl)indoline,
(S)-2-(cis-3,5.-dimethylpiperidin-1-yl)carbonyl-1-(2-pyrimidinyl)indoline,
(S)-1-(1-methyl-6-oxo-1,6-dihydropyridazin-3-yl)-2-[[(S)-3-methylpiperidin-1-yl]carbonyl]indoline,
((S)-1-(1-methyl-6-oxo-1,6-dihydropyridazin-3-yl)-2-[(cis-3,5-dimethylpiperidin-1-yl)carbonyl]indoline,
(S)-2-(cis-3,5-dimethylpiperidin-1-yl)carbonyl-1-(3-pyridazinyl)indoline,
(S)-2-[(S)-3-methylpiperidin-1-yl]carbonyl-1-(2-pyrazinyl)indoline,
(S)-2-(cis-3,5-dimethylpiperidin-1-yl)carboriyl-1-(2-pyrazinyl)indoline,
(S)-2-[(S)-3-methylpiperidin-1-yl]carbonyl-1-(3-pyridazinyl)indoline,
(S)-1-(1-ethyl-6-oxo-1,6-dihydropyridazin-3-yl)-2-[[(S)-3-methylpiperidin-1-yl]carbonyl]indoline,
(S)-1-(1-methyl-6-oxo-1,6-dihydropyridazin-3-yl)-2-[(1-perhydroazepinyl)carbonyl]indoline,
(S)-1-(1-methyl-6-oxo-1,6-dihydropyridazin-3-yl)-2-[(1-perhydroazocinyl)carbonyl]indoline,
(S)-1-(3-chloro-1,2,4-triazolo[4,3-b]pyridazin-6-yl)-2-[[(S)-3-methylpiperidin-1-yl]carbonyl]indoline;
(S)-1-[1=(2-fluoroethyl-)-6-oxo-1,6-dihydropyridazin-3-yl]-2-[[(S)-3-methylpiperidin-I-yl]carbonyl]indoline,
(S)-2-[[(S)-3-methylpiperidin-l-yl]carbonyl]-1-(3-methyl-1,2,4-triazolo[4,3-b]pyridazin-6-yl)indoline,
(S)-2-[[(S)-3-methylpiperidin-1-yl]carbonyl]-1-(1,2,4-triazolo[4,3-b]pyridazin-6-yl)indoline, and
(S)-2-[[(S)-3-methylpiperidin-1-yl]carbonyl]-1-(6-oxo-1,6-dihydropyridazin-3-yl)indoline; or a pharmaceutically acceptable acid addition salt thereof.

10. A pharmaceutical composition comprising a 2-(cyclic aminocarbonyl)indoline derivative or a pharmaceutically acceptable acid addition salt thereof according to any one of claims 1-9.

11. A medicament for treating anxiety disorder or depression comprising as an active component a 2-(cyclic aminocarbonyl) indoline derivative or a pharmaceutically acceptable acid addition salt thereof according to any one of claims 1-9.

12. Use of a 2-(cyclic aminocarbonyl)indoline derivative or a pharmaceutically acceptable acid addition salt thereof according to any one of claims 1-9, for treating anxiety disorder or depression.

13. A method for treating anxiety disorder or depression comprising administering to a patient in need thereof a therapeutically effective amount of a 2-(cyclic aminocarbonyl)indoline derivative or a pharmaceutically acceptable acid addition salt thereof according to one of claims 1-9.
